(19) Europäisches Patentamt
European Patent Office
Office européen des brevets

(11) EP 4 163 255 A1

(12) EUROPÄISCHE PATENTANMELDUNG

(43) Veröffentlichungstag:
**12.04.2023 Patentblatt 2023/15**

(21) Anmeldenummer: **21201336.1**

(22) Anmeldetag: **06.10.2021**

(51) Internationale Patentklassifikation (IPC):
***C01B 32/80*** (2017.01) ***C01B 3/22*** (2006.01)
***C01B 3/26*** (2006.01) ***C07C 1/04*** (2006.01)
***C10K 3/02*** (2006.01) ***C07C 263/10*** (2006.01)

(52) Gemeinsame Patentklassifikation (CPC):
(C-Sets verfügbar)
**C01B 32/80; C01B 3/22; C01B 3/26; C07C 29/151**

(Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude K12**
**51365 Leverkusen (DE)**

(54) **VERFAHREN ZUR HERSTELLUNG VON PHOSGEN**

(57) Es wird ein Verfahren zur Herstellung von Phosgen aus Chlor und Kohlenmonoxid (1) beschrieben, das sich dadurch kennzeichnet, dass durch katalytische Zersetzung von Methanol (4) erhaltenes Kohlenmonoxid bereitgestellt und dieses bereitgestellte Kohlenmonoxid (2) mit Chlor zu Phosgen (3) umgesetzt wird. Mit Hilfe dieses Verfahrens gelingt es, dass trotz möglicher Schwankungen in der Bereitstellung erneuerbarer Energien ein emissionsarmes Phosgen für die Herstellung von Isocyanaten und Polycarbonaten bereitgestellt werden kann. Methanol dient als Quelle für Kohlenmonoxid, wobei das dafür genutzte Methanol vorzugsweise unter Einsatz von regenerativer Energie aus Wasserstoff und $CO_x$ mit x = 1 oder 2 bereitgestellt wird.

Fig.1

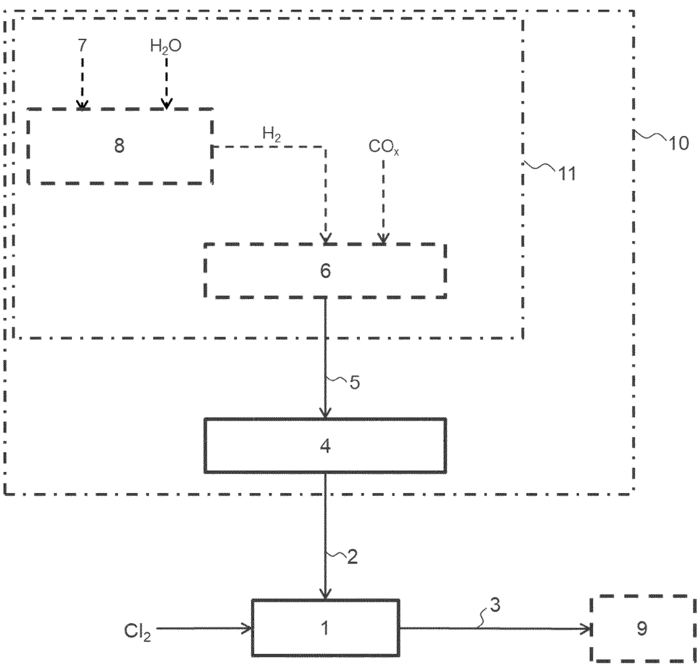


EP 4 163 255 A1

(52) Gemeinsame Patentklassifikation (CPC): (Forts.)

C-Sets
**C07C 29/151, C07C 31/04**

## Beschreibung

Die vorliegende Erfindung betrifft ein Verfahren, in dem Verwertungsprodukte, insbesondere Synthesegas, aus Methanol gewonnen und zur Bereitstellung als Reaktand für die chemische Produktion von Phosgen eingesetzt wird, welches wiederum, beispielsweise chemisch, zu organischem Isocyanat oder zu Polycarbonat umgesetzt werden kann.

Die großtechnische Herstellung von Di- und Polyisocyanaten durch Umsetzung der korrespondierenden organischen Amine mit Phosgen ist seit längerem aus dem Stand der Technik bekannt, wobei die Reaktion in der Gas- oder Flüssigphase sowie diskontinuierlich oder kontinuierlich durchgeführt werden kann (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise Ullmanns Encyklopädie der technischen Chemie, 4. Aufl. (1977), Band 13, S. 351 bis 353 sowie G. Wegener et. al. Applied Catalysis A: General 221 (2001), p. 303 - 335, Elsevier Science B.V. Es kommen dabei sowohl aromatische Isocyanate wie beispielsweise Methylendiphenyldiisocyanat (MMDI - "monomeres MDI"), Polymethylen-Polyphenylen-Polyisocyanate (das sind die höheren Homologen des MMDI, auch PMDI, "polymeres MDI" genannt; diese fallen technisch stets im Gemisch mit MMDI-Anteilen an) oder Toluylendiisocyanat (TDI) als auch aliphatische Isocyanate wie z. B. Hexamethylendiisocyanat (HDI) oder Isophorondiisocyanat (IPDI) weltweit zum Einsatz.

Die großtechnische Herstellung von Polycarbonaten erfolgt überwiegend durch Umsetzung eines Diphenols oder mehreren verschiedenen Diphenolen und einem Carbonylhalogenid nach dem Phasengrenzflächenverfahren. Die Polycarbonatherstellung nach dem Phasengrenzflächenverfahren ist bereits von Schnell, "Chemistry and Physics of Polycarbonates", Polymer Reviews, Volume 9, Interscience Publishers, New York, London, Sydney 1964, S. 33-70; D. Freitag, U. Grigo, P.R. Müller, N. Nouvertne', BAYER AG, "Polycarbonates" in Encyclopedia of Polymer Science and Engineering, Volume 1 1, Second Edition, 1988, S. 651-692.

Des Weiteren wird auch in EP 0 517 044 A2 oder EP 520 272 A2 das Phasengrenzflächenverfahren zur Herstellung von Polycarbonat beschrieben.

Zur Herstellung von Polycarbonat nach dem Phasengrenzflächenverfahren wird ein in wässrig alkalischer Lösung oder Suspension vorgelegtes Dinatriumsalz eines Diphenols oder ein in wässrigalkalischer Lösung oder Suspension vorgelegtes Gemisch mehrerer verschiedener Diphenole in Gegenwart eines inerten organischen Lösungsmittels oder Lösungsmittelgemisches mit einem Carbonylhalogenid, insbesondere mit Phosgen, umgesetzt.

Die großtechnische Herstellung von Phosgen, das bei der Phosgenierung der entsprechenden organischen Amine zum Einsatz kommt, aus CO und Chlor an Aktivkohle-Katalysatoren in einem Rohrbündelreaktor ist ebenfalls aus dem Stand der Technik bekannt (z.B. Ullmann's Encyclopedia of Industrial Chemistry, 5th ed. Vol. A 19p 413f., VCH Verlagsgesellschaft mbH, Weinheim, 1991). Dabei wird Kohlenmonoxid im stöchiometrischen Überschuss mit Chlor vereinigt und über einen Festbettkatalysator geleitet. Als Katalysator wird für industrielle Zwecke Aktivkohle eingesetzt, wobei die Auswahl einer geeigneten Aktivkohle bis heute empirisch erfolgt (Mitchell et al.: Selection of carbon catalysts for the industrial manufacture of phosgene; Catal. Sci. Technol., 2012, 2, 2109-2115).

Die großtechnische Herstellung der für die Isocyanatherstellung notwendigen organischen Amine erfolgt üblicherweise durch Hydrierung der entsprechenden organischen Nitroaromaten mit Wasserstoff unter Verwendung eines Katalysators. Beispielsweise wird in der Druckschrift EP 1 882 681 A ein Verfahren zur adiabaten Hydrierung von Nitroaromaten zu aromatischen Aminen in der Gasphase an ortsfesten Katalysatoren beschrieben, worin der umzusetzende Nitroaromat mit Wasserstoff, Wasser, ggf. Stickstoff sowie im Wesentlichen in Abwesenheit von dem aus dem Nitroaromaten hergestellten aromatischen Amin unter Druck bei erhöhter Temperatur über den Katalysator geleitet wird.

Die Herstellung von Chlor erfolgt technisch durch die Umsetzung von HCl (Elektrolyse oder katalytische Umsetzung) oder NaCl (Elektrolyse). HCl-Recyclingverfahren sind Stand der Technik (vgl. Ullmann Enzyklopädie der technischen Chemie, Weinheim, 4. Auflage 1975, Bd. 9, S. 357 ff. oder Winnacker-Küchler: Chemische Technik, Prozesse und Produkte, Wiley-VCH Verlag, 5. Auflage 2005, Bd. 3, S. 512 ff.): Dazu zählen insbesondere die Elektrolyse von Salzsäure sowie die Oxidation von Chlorwasserstoff. Das von Deacon 1868 entwickelte Verfahren der katalytischen Chlorwasserstoffoxidation mit Sauerstoff in einer exothermen Gleichgewichtsreaktion stand am Anfang der technischen Chlorchemie:

$$4\ HCl + O_2 \leftrightarrows 2Cl_2 + 2\ H_2O$$

Durch die Elektrolyse von NaCl (Chloralkaliprozess) wurde das Deacon-Verfahren jedoch stark in den Hintergrund gedrängt, aber dennoch technisch angewendet. Nahezu die gesamte Produktion von Chlor erfolgte durch Elektrolyse wässriger Kochsalzlösungen (Ullmann Encyclopedia of industrial chemistry, seventh release, 2006).

Wasserstoff sowie Kohlenmonoxid werden heutzutage größtenteils durch den Dampfreformierungsprozess erzeugt. Dabei werden Kohlenwasserstoffe mittels Wasserdampf unter Einsatz eines heterogenen Katalysators zu den Produkten Wasserstoff und Kohlenmonoxid umgesetzt. Als Ausgangsmaterial wird dabei größtenteils Erdgas verwendet, wobei auch Hüttengase, Reststoffe (wie z.B. Plastikverpackungsmüll, Matratzen oder andere polymere Reststoffe) oder

Naptha eingesetzt werden können.

Die endotherme Reaktion (Prozesstemperatur: 800-900°C), welche typischerweise in einem Rohrbündelreaktor befüllt mit einem Nickelkatalysator durchgeführt wird, setzt sich aus folgenden Prozessen/Reaktionen zusammen:

$$CH_4 + H_2O \leftrightarrows CO + 3H_2$$

$$CO + H_2O \leftrightarrows CO_2 + H_2$$

Die Zusammensetzung des Produktgases hängt dabei stark von den Prozessparametern ab. Wichtigste Parameter sind hierbei Druck, Reform Gas Temperatur sowie das Verhältnis zwischen Dampf und Kohlenwasserstoffen.

Bezüglich der Verwendung von Erdgas ist hinzuzufügen, dass bevor jenes im Prozess eingesetzt werden kann eine Aufreinigung erfolgen muss. Hierbei müssen nicht nur Schwersieder abgetrennt werden sondern auch mögliche Katalysatorgifte aus dem Feedgas entfernt werden. Essentiell ist hier die Abtrennung von Schwefel, welcher ein besonders starkes Katalysatorgift darstellt. Neben Erdgas können auch höhere Kohlenwasserstoffe genutzt werden. Dafür sind jedoch Prereformierungsschritte notwendig.

Bei der Synthesegasgewinnung kann durch die Kombination von mehreren Verfahren eine energetische Kopplung erzielt werden. Hier wird der endotherme Dampfreformierungsprozess

$$CH_4 + H_2O \leftrightarrows CO + 3H_2$$

an die exotherme partielle Oxidation (Teilverbrennung von Kohlenwasserstoffen, welche ein $H_2$ reiches Synthesegas ergibt; siehe ULLMANN'S Encyclopedia of Industrial Chemistry: "Gas production", 2016.) gekoppelt, sodass der Dampfreformer mit dem heißen Austrittsgas der partiellen Oxidation beheizt. Eine solche Kombination aus den benannten Verfahren wurde bspw. in US6730285B2 beschrieben.

Je nach Verwendungszweck wird das gewonnen Synthesegas weiteren Behandlungsschritten unterzogen. So wird bspw. die Water-Gas-Shift Reaktion technisch genutzt um aus vorhanden $CO/H_2$ Mischungen den Wasserstoffanteil zu erhöhen. Dabei wird CO mit $H_2O$ über einem heterogenen Katalysator umgesetzt:

$$CO + H_2O\ CO_2 + H_2$$

Dieser Prozess kann dabei bei höheren (350-400°C) als auch milderen Bedingungen (200-250°C) genutzt werden.

Ein weiteres Verfahren zur Herstellung eines CO-haltige Synthesegases stellt die elektrochemische $CO_2$ Reduktion dar. Hierbei kann die $CO_2$-Elektrolyse beispielsweise eine Hochtemperaturelektrolyse sein, die bei einer Temperatur von mehr als 600°C ggf. auch unter Zusatz von Wasser zur Herstellung von Synthesegas, betrieben wird. Hochtemperaturelektrolysen sind grundsätzlich bekannt und im Markt verfügbar z.B. von Haldor Topsoe, eCOs™. Bei der Hochtemperaturelektrolyse entsteht an der Anode ebenfalls Sauerstoff.

Im Falle der Niedertemperaturelektrolyse wird $CO_2$ insbesondere an einer Gasdiffusionselektrode zu Kohlenmonoxid und ggf. Wasserstoff umgesetzt. Gleichzeitig kann an der Anode $O_2$ oder ggf. alternativ auch Chlor erzeugt werden. Das Verfahren ist beispielhaft in T. Haas et. al., Nature Catalysis 2018, 1, 32-39 beschrieben.

Nach den bekannten Prinzipien kann bei der Niedertemperatur-Elektrolyse auch ein MEA (Membran-Electrode-Assembly) Konzept zum Einsatz kommen. Hierbei wird auf die Membran ein Katalysator aufgebracht. Eine davor gelagerte Gasdiffusionsschicht reguliert den Gas- und Flüssigkeitstransport. Dies kann sowohl auf der Anoden- als auch auf der Kathodenseite erfolgen. Weiterhin besteht die Möglichkeit eine Gasdiffusionselektrode in direktem Kontakt mit der Membran zu bringen.

Ein weiteres Verfahren zur Herstellung eines CO-haltige Synthesegases stellt die sog. Reverse Water Gas Shift Reaktion (RWGS) dar.

Je nach nachfolgendem Produktionsschritt sind die Synthesegasbestandteile weiter aufzureinigen. So müssen bspw. für die Ammoniaksynthese sämtliche $CO_2$ Anteile entfernt werden, was üblicherweise durch Scrubbing-Prozesse erfolgt. Spuren von CO werden hier über Methanisierung oder N2-scrubbing entfernt. Zusätzlich können je nach Bedarf auch Druckwechseladsorptionen zur weiteren Aufreinigung eingesetzt werden.

Die großtechnische Herstellung von Methanol erfolgt heutzutage fast ausschließlich durch das sog. Niederdruckverfahren, welches in Druckbereichen zwischen 50 und 120 bar arbeiten. Verglichen mit den historischen Hochdruckverfahren, welche bei 250-400 bar operieren, konnten so die Produktions- und Investitionskosten drastisch gesenkt werden. Die Verringerung des Prozessdruckes wurde letztendlich durch den Einsatz von Cu/ZnO-Katalysatoren anstelle von $ZnO/Cr_2O_3$-Katalysatoren ermöglicht. Dieser Wechsel wurde vor allem durch Fortschritte in der Prozessführung (u.a. Verringerung möglicher Katalysatorgifte im Feed) bewirkt. Vorteil des Cu/ZnO-Katalysators ist hier vor allem, dass die Synthese bei wesentlichen geringen Temperaturen (200-300 °C) durchgeführt werden kann, wodurch letztendlich

niedrigere Systemdrücke benötigt werden.

Für die Methanolsynthese werden Kohlenstoffoxide zusammen mit Wasserstoff (Synthesegas) umgesetzt. Dieser Prozess ist exotherm und kann durch folgende Reaktionsgleichungen beschrieben werden:

$$CO + 2H_2\ CH_3OH$$

$$CO_2 + 3H_2\ CH_3OH + H_2O$$

Beide Reaktionen werden durch die ebenfalls exotherme Water-Gas-Shift Reaktion gekoppelt, welche wie folgt beschrieben werden kann:

$$CO + H_2O\ CO_2 + H_2$$

Üblicherweise wird für die Methanolsynthese hauptsächlich aus CO und $H_2$ bestehendes Synthesegas eingesetzt, dessen Zusammensetzung starken Einfluss auf die optimale Nutzung haben. Gemäß D1 kann die Zusammensetzung über die Stöchiometriezahl (SZ) beschrieben werden:

$$SZ = \frac{n_{H_2} - n_{CO_2}}{n_{CO} + n_{CO_2}}$$

Für SZ = 2 liegen die Reaktanden im stöchiometrischen Verhältnis gemäß der o.g. Reaktionsgleichungen vor. Real werden jedoch leicht höhere Werte für SZ (2,01-2,1) verwendet, wobei dies durch einen höheren Wasserstoffanteil im Synthesegas realisiert wird (Dittmeyer et al. "Chemische Technik", Band 4, 5. Auflage). Trotz dieser optimierten Zusammensetzung ist ein Komplettumsatz jedoch aus thermodynamischer Sicht nicht möglich. Eine Rückführung der nicht umgesetzten Edukte ist daher zwingend notwendig.

Bedingt durch die im Reaktor anfallenden Prozesse muss das aus dem Reaktor austretende Produkt weiter aufgereinigt werden. So sind z.B. signifikante Mengen Wasser, aber auch in geringen Mengen weitere Nebenprodukte wie Dimethylether, Methylformiat oder höhere Alkohole enthalten. Die Auftrennung des Produktgemisches erfolgt dabei üblicherweise durch Destillation.

Hinsichtlich technologischer Aspekte gilt die Methanolsynthese als weitestgehend optimiert. Dennoch können bspw. durch die Verbesserung des Energieaustausches zwischen den einzelnen Prozessstufen weitere Optimierung erzielt werden. Bspw. kann bei der Synthesegasgewinnung durch die Kombination von mehreren Verfahren eine energetische Kopplung erzielt werden. Hier wird der endotherme Dampfreformierungsprozess

$$CH_4 + H_2O\ CO + 3H_2$$

an die exotherme partielle Oxidation (Teilverbrennung von Kohlenwasserstoffen, welche ein $H_2$ reiches Synthesegas ergibt; siehe US 6340437 B1) gekoppelt, sodass der Dampfreformer mit dem heißen Austrittsgas der partiellen Oxidation beheizt. Eine solche Kombination aus den benannten Verfahren wurde bspw. in US 6730285 B2 beschrieben.

Während Methanol in nahezu allen technischen betriebenen Anlagen durch die Umsetzung von CO und $H_2$ hergestellt wird, wird der Einsatz von $CO_2$ (s.o.) als Rohstoff (siehe bspw. ChemCatChem 2019, 11, 4238—4246) immer intensiver verfolgt. ChemCatChem 2019, 11, 4238—4246 beschreibt die Gleichgewichtsausbeute bei 230°C und 50bar mit 30%.

Die Herstellung von Synthesegas durch die Zersetzung von Methanol ist ein bekanntes und kommerzielles Verfahren. Das Verfahren kann als Rückreaktion der oben beschriebenen technischen Methanol Herstellung gesehen werden, da die Umsetzung über das gleiche chemische Gleichgewicht abläuft. Dabei ist jedoch bekannt (Topics in Catalysis Vol. 22, Nos. 3—4, April 2003), dass sich trotz gleicher Netto-Reaktionsgleichung der Mechanismus der Rückreaktion von der Methanolsynthese unterscheidet. In Abwesenheit von Wasser wird hier unter Freisetzung von Wasserstoff zunächst Methyl-Formiat gebildet, welches anschließend zu CO und $H_2$ zerfällt:

$$2CH_3OH \rightarrow CH_3OCHO + 2H_2$$

$$CH_3OCHO \rightarrow 2CO + 2H_2$$

In Anwesenheit von Wasser bildet die Reaktion die Grundlage für den allgemein bekannten Methanol-Reformer. Zielprodukte sind hier analog zur Water-Gas-Shift Reaktion $CO_2$ und $H_2$:

$CH_3OH + H_2O\ CO_2 + 3H_2$

US 6583084 beschreibt die Herstellung von Wasserstoff durch die Zersetzung von Methanol in der Gasphase. Es werden Katalysatoren auf Cu-basis verwendet.

EP0409517 beschreibt ein Verfahren zur Herstellung von Synthesegas aus Methanol. Als Katalysatoren werden hier Materialien auf Cr- und Zn-Basis eingesetzt. Der Katalysator kann dabei mit verschiedenen Materialien dotiert werden. Die Zersetzung findet bei einer Temperatur zwischen 270°C und 400°C und einem Druck von 20 kg $cm^{-2}$ (entspricht ca. 20 bar) statt. Die Reaktion wird in einem Rohrbündelreaktor durchgeführt.

US 20050108941 A beschreibt ein Verfahren zur Herstellung von Wasserstoff oder Synthesegas durch Umsetzung von Methanol in einer Suspension. Dabei wird ein Katalysator bestehend aus Kupfer, Zinkoxid, Aluminium und Chrom eingesetzt.

US 6699457 beschreibt ein Verfahren zur Herstellung von Wasserstoff oder Synthesegas durch Umsetzung von Methanol in einer Suspension oder in der Gasphase. Als aktive Katalysatorkomponente wird hier ein Gruppe VIII Übergangsmetall eingesetzt. Der Prozess wird hier bspw. bei einer Temperatur unter 400°C durchgeführt, wobei der Druck im System so angepasst wird, dass Wasser und der eingesetzte Kohlenwasserstoff ("oxygenated hydrocarbon") als Flüssigkeiten vorliegen.

Es kann zusammengefasst werden, dass neben der Wahl des Katalysators auch die Anwesenheit von Wasser einen signifikanten Einfluss auf die Selektivität des Prozesses hat. Durch die Anwesenheit von Wasser kann bspw. der Anteil CO im Synthesegas gesenkt werden.

Ein weiteres Verfahren zur Herstellung von Wasserstoff stellt die elektrochemische Umwandlung von Wasser dar. Unter Nutzung von Strom wird hier Wasser in Wasserstoff und Sauerstoff gespalten. Bekannte Verfahren sind die Protonen-Austausch-Elektrolyse (PEM), welche bspw. in EP 3489394 A beschrieben wird. Ein weiteres Verfahren stellt die alkalische Wasserelektrolyse dar. Beide Verfahren unterscheiden sich durch insbesondere durch die Verfahrensparameter wie bspw. Temperatur, Druck und pH-Wert. Beide Verfahren führen jedoch zu den Zielprodukten Wasser- und Sauerstoff.

Es war die Aufgabe der vorliegenden Erfindung ein Verfahren zur Herstellung von Phosgen bereitzustellen, mit dessen Hilfe trotz möglicher Schwankungen in der Bereitstellung erneuerbarer Energien ein emissionsarmes Phosgen für die Herstellung von Isocyanaten und Polycarbonaten bereitgestellt werden kann.

Ein erster Gegenstand der Erfindung ist ein Verfahren zur Produktion von Phosgen aus Chlor und Kohlenmonoxid, worin durch katalytische Zersetzung von Methanol erhaltenes Kohlenmonoxid bereitgestellt und das bereitgestellte Kohlenmonoxid mit Chlor zu Phosgen umgesetzt wird.

Für die Bereitstellung des Kohlenmonoxids ist es im Sinne der Erfindung ausreichend, wenn das Kohlenmonoxid ein Verfahrensprodukt einer katalytischen Zersetzung von Methanol ist. Dies bedeutet, dass es bei Durchführung des erfindungsgemäßen Verfahrens für die Ausführung des Schrittes der Bereitstellung des Kohlenmonoxids bereits ausreicht, das besagte Kohlenmonoxid lediglich im Rahmen einer Belieferung als Rohstoff aus einem Lagerbehälter oder aus einer Zuleitung zu entnehmen. In diesem Falle führt der Phosgen-Produzent als Ausführender des erfindungsgemäßen Verfahrens die katalytische Zersetzung von Methanol zur Herstellung des Kohlenmonoxids nicht selbst aus, sondern er sorgt nur dafür, dass das bereitgestellte Kohlenmonoxid durch einen Lieferanten durch katalytische Zersetzung von Methanol hergestellt wurde und deren Verfahrensprodukt ist.

Ebenso ist es im Rahmen einer Ausführungsform erfindungsgemäß möglich, wenn zur Bereitstellung des Kohlenmonoxids vorgenannte katalytische Zersetzung des Methanols als integrale Schritte eines erfindungsgemäßen Verfahrens zur Herstellung von Phosgen durch den Phosgen-Produzenten selbst ausgeführt werden und das dabei erhaltene Kohlenmonoxid der Phosgenherstellung zugeführt wird.

Diese Möglichkeiten der Bereitstellung, i.e. Belieferung oder eigene Herstellung durch den Phosgen-Produzenten, gelten jeweils auch für die nachfolgend genannten Ausführungsformen der Bereitstellung des Kohlenmonoxids.

In dem erfindungsgemäßen Verfahren wird das für den Einsatz in der Phosgenherstellung bereitgestellte Kohlenmonoxid aus Methanol hergestellt. Eine bevorzugte Ausführungsform des Verfahrens ist dadurch gekennzeichnet, dass dafür vorab Methanol bereitgestellt wird, welches Verfahrensprodukt einer elektrochemischen Reaktion, einer homogen katalysierten Reaktion oder einer heterogen katalysierten Reaktion ist.

Für die Bereitstellung des Methanols ist es demnach ausreichend, wenn das Methanol ein Verfahrensprodukt mindesten einer der vorgenannten Reaktionen ist. Dies bedeutet, dass es für die Ausführung des Schrittes der Bereitstellung des Methanols bereits ausreicht, das besagte Methanol lediglich im Rahmen einer Belieferung als Rohstoff aus einem Lagerbehälter oder aus einer Zuleitung zu entnehmen. In diesem Falle führt der Phosgen-Produzent als Ausführender des erfindungsgemäßen Verfahrens die vorgenannte Reaktion zur Herstellung des Methanols nicht selbst aus, sondern er sorgt nur dafür, dass das zur Kohlenmonoxid-Bereitstellung für die katalytische Zersetzung von Methanol bereitgestellte Methanol durch Anwendung mindestens einer dieser besagten Methoden hergestellt wurde und somit deren Verfahrensprodukt ist.

Ebenso ist es erfindungsgemäß möglich, wenn zur Bereitstellung des Methanols mindestens eine der vorgenannten Reaktionen zur Herstellung des Methanols als integrale Schritte eines erfindungsgemäßen Verfahrens zur Herstellung von Phosgen durch den Phosgen-Produzenten ausgeführt werden und das dabei erhaltene Methanol, gegebenenfalls nach einer Zwischenlagerung in einem Lagerbehälter, der katalytischen Zersetzung des Methanols unter Bereitstellung von Kohlenmonoxid zugeführt wird.

Die Möglichkeiten der Bereitstellung, i.e. Belieferung oder eigene Herstellung des speziellen Methanols durch den Phosgen-Produzenten, gelten jeweils auch für die nachfolgend genannten Ausführungsformen der Bereitstellung des Methanols.

Besonders bevorzugt ist eine Ausführungsform, worin das für das erfindungsgemäße Verfahren bereitzustellende Methanol unter Verwendung regenerativer Energie hergestellt wurde, vorzugsweise an einem geografischen Ort mit guter Verfügbarkeit von regenerativer Energie, um anschließend entweder dieses Methanol durch Lieferung per Transport in Behältern oder per Methanolstrom im Rahmen eines kontinuierlichen Prozesses für das erfindungsgemäße Verfahren bereitzustellen. Für die Bereitstellung im Rahmen eines kontinuierlichen Prozesses steht die Methanolherstellung bevorzugt in Fluidverbindung mit der Methanolzersetzung, z.B. über eine Rohrleitung.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus.

Wird regenerative Energie im Rahmen der vorliegenden Erfindung als Energiequelle, insbesondere für die Bereitstellung von elektrischem Strom, genutzt, dann kann es für die Gewährleistung einer Ausführung des erfindungsgemäßen Verfahrens in einer Mangelperiode von regenerativer Energie vorteilhaft sein, zu einem Zeitpunkt der Verfügbarkeit von regenerativer Energie diese zur Überbrückung von Mangelperioden zu speichern, z.B. in Form von elektrischem Strom in einem Akku.

Eine "elektrochemische Reaktion" findet erfindungsgemäß durch Anlegen von elektrischem Strom im Reaktionsmedium (z.B. über mindestens eine in das Reaktionsmedium eintauchende Elektrode) in Gegenwart mindestens eines Reaktanden (z.B. Kohlendioxid oder Methanol) statt.

Eine "katalysierte Reaktion" oder "katalytische Umsetzung" findet erfindungsgemäß unter Einsatz eines Katalysators statt, der die Produktbildung aus mindestens einem Reaktanden (z.B. Kohlendioxid oder Methanol) katalysiert. Eine "homogen katalysierte Reaktion" findet erfindungsgemäß unter Einsatz eines homogenen Katalysators und eine "heterogen katalysierte Reaktion" unter Einsatz eines heterogenen Katalysators statt.

Erfindungsgemäß bevorzugt wird das für die Zersetzung bereitgestellte Methanol aus einem Gasstrom hergestellt, der mindestens $CO_x$ mit x = 1 oder 2 und gegebenenfalls Wasserstoffgas enthält.

Im Rahmen einer besonders bevorzugten Ausführungsform des Verfahrens wird das für die Zersetzung bereitgestellte Methanol aus einem Kohlenmonoxid und Wasserstoff haltigem Gasstrom, der durch eine Methode oder einer Kombination von Methoden aus der Reihe Dampfreformierung von Methan, partielle Oxidation von Kohlenwasserstoffen, Vergasung von Biomasse, Elektrolyse, Reverse Water Gas Shift erhalten wird, hergestellt. Besonders bevorzugt ist mindestens eine Methode eine elektrochemische Reaktion, eine homogen katalysierte Reaktion oder eine heterogen katalysierte Reaktion.

Es hat sich weiter als bevorzugt herausgestellt, wenn in einer Ausführungsform des Verfahrens Methanol durch die Umsetzung von $CO_2$ mit oder ohne Wasserstoff gewonnen und für die katalytische Zersetzung bereitgestellt wird. Dabei ist es wiederum besonders bevorzugt, wenn die Umsetzung eine elektrochemische Reaktion, eine homogen katalysierte Reaktion oder eine heterogen katalysierte Reaktion ist. Das dafür eingesetzte $CO_2$ stammt in einer weiteren Ausführungsform zusätzlich aus einer weiteren $CO_2$-Quelle, beispielsweise das bei der Bereitstellung von Wärmeenergie emittierte $CO_2$ oder das $CO_2$ aus einer externen $CO_2$-Quelle, z.B. einem Industrieabgas.

Als eine bevorzugte $CO_2$-Quelle dient mindestens eine externe $CO_2$-Quelle, die $CO_2$ beiträgt, welches nicht durch das erfindungsgemäße Verfahren emittiert wird. Eine externe $CO_2$ Quelle wäre z.B. das $CO_2$, das bei einer Zementherstellung, bei einer $H_2$-Herstellung für die Ammoniaksynthese anfällt, bei der Fermentation anfällt, bei Verbrennung von Brennstoffen (z.B. einer Abfallverbrennung) im Abgas entsteht, oder $CO_2$, das aus der Luft gewonnenen wird. Dieses $CO_2$ aus einer externen $CO_2$-Quelle wird im Rahmen einer bevorzugten Ausführungsform des erfindungsgemäßen Verfahrens durch Absorption eines $CO_2$-Anteils aus (i) Prozessgasen oder Abgasen, die aus mindestens einem Prozess ausgewählt aus Zementherstellung, Fermentation, $H_2$-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu $CO_2$ und Kalilauge thermisch zersetzt werden kann. Das dabei freigesetzte $CO_2$ wird dann dem erfindungsgemäßen Reformerprozess zur Synthese von Kohlenmonoxid zugeführt. Zur Bereitstellung der für die $CO_2$ Freisetzung notwendigen Wärmeenergie kann beispielsweise die erzeugte Wärmeenergie aus dem besagten Reformerprozess eingesetzt werden.

Das für die Zersetzung genutzte Methanol wird in einer besonders bevorzugten Ausführungsform des Verfahrens aus Kohlendioxid hergestellt, insbesondere (i) durch eine katalytische Umsetzung eines Synthesegases, enthaltend mehr als 50 Vol.% einer Mischung aus Kohlendioxid und Wasserstoff, (ii) durch Fermentation oder (iii) durch elektrochemische Umwandlung von Kohlendioxid hergestellt. Dabei wird jedem Syntheseprozess (i) bis (iii) ein $CO_2$ haltiger Stoffstrom als Feed zugeführt. Im Falle der Fermentation (ii) und insb. der katalytischen Umsetzung (i) enthält der Feed zudem Wasserstoff. Besonders bevorzugt wird der für die Schritte (i) oder (ii) benötigte Wasserstoff durch mindestens ein Verfahren, ausgewählt aus der Elektrolyse von Wasser, der partiellen Oxidation oder der Biomassevergasung, oder Mischungen davon hergestellt. Dabei ist es wiederum ganz besonders bevorzugt, wenn für die Elektrolyse von Wasser elektrischer Strom aus regenerativer Energie genutzt wird, insbesondere ausgewählt aus Sonnenenergie, Windkraft, Wasserkraft oder Mischungen daraus. Dieser Strom aus regenerativer Energie kann auch aus einem Speichermedium wie z.B. einem Akku entnommen werden.

Die Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

In einer weiteren Ausführung wird dem Synthesegas für die katalytische Umwandlung Kohlenmonoxid und/oder Wasser als Nebenkomponente zugesetzt. Weitere Bestandteile können Inertgase sein.

Für die elektrochemische Umwandlung (iii) wird ein Gasstrom enthaltend mehr als 50 Vol.% einer Mischung von Kohlendioxid und Wasserdampf bevorzugt.

In allen zuvor genannten Prozessen (i) bis (iii) wird ein Methanol-haltiges Rohprodukt erhalten, welches bevorzugt einer Aufbereitung unterzogen wird. Bei der Aufarbeitung werden zunächst $CO_2$, CO und $H_2$ abgetrennt. Bevorzugt wird diese Fraktion in die Methanolsynthese zurückgeführt. Ganz besonders bevorzugt wird ein Teil des Gases ausgeschleust (Purge). Nach der Abtrennung wird das verbleibende Gemisch einer Destillation bzw. Rektifikation unterzogen. Hierbei wird als Zielprodukt Methanol erhalten, welches bevorzugt aus der Aufarbeitung in ein Methanolreservoir geleitet wird. Weitere aus der Destillation abgetrennte Komponenten können u.a. Dimethylether und Ethanol sein. Als weitere Fraktion wird bei der Synthese Wasser erhalten, welches entweder der Entsorgung oder bevorzugt der o.g. Wasserelektrolyse zugeführt wird.

Im katalytischen Zersetzungsschritt des erfindungsgemäßen Verfahrens wird bereitgestelltes Methanol (beispielsweise zur Bereitstellung aus einem Lagerbehälter entnommen oder im Rahmen eines kontinuierlichen Verfahrens vorab durch mindestens einen Prozess (i) bis (iii) hergestellt) im Rahmen einer bevorzugten Ausführungsform durch Inkontaktbringen von gasförmigem Methanol mit einem Katalysator und katalytisch unter Bildung von Kohlenmonoxid und gegebenenfalls zusätzlich von Wasserstoffgas zersetzt wird. Sofern das Methanol zu diesem Zweck nicht gasförmig vorliegt (z.B. bei Entnahme aus einem Lagerbehälter) wird dieses vorab einer Verdampfung unterworfen. Der für die Verdampfung notwendige Energieeintrag kann durch Nutzung von fossilen Brennstoffen, wie z.B. Erdgas, als Energiequelle, erzeugt werden. Dem erfindungsgemäßen Verfahren wird für die Einstellung der zur Verdampfung notwendigen Temperatur besonders bevorzugt Wärmeenergie zugeführt, die mindestens durch eine Methode bereitgestellt wird, ausgewählt aus (i) der Verbrennung von Brennstoff, der mittels regenerativer Energie erzeugten Wasserstoff (gasförmiges $H_2$) enthält, (ii) der Verbrennung von Brennstoff, der Methan aus biologischer Quelle enthält oder (iii) der Umwandlung von aus regenerativer Energie erzeugter, elektrischer Energie in Wärme. Dabei ist es wiederum besonders bevorzugt, wenn als regenerative Energie wahlweise Windkraft, Sonnenenergie, Wasserkraft oder Mischungen daraus, eingesetzt wird.

Bevorzugt wird zur katalytischen Zersetzung gasförmiges Methanol über einen Katalysator, dessen Aktivkomponente mindestens eine Übergangsmetall-Spezies enthält, geleitet. Als Übergangsmetall-Spezies versteht der Fachmann alle d-Block Elemente und deren chemische Verbindungen. Besonders bevorzugt handelt es sich bei dem Katalysator um eine Übergangsmetall-Spezies, welche auf einen Träger aufgebracht ist. Ganz besonders bevorzugt handelt es sich dabei um mindestens eine Übergangsmetall-Spezies ausgewählt aus der Gruppe VII, VIII oder XI des Periodensystems der Elemente, welche wiederum bevorzugt auf einen Träger aufgebracht ist.

Die katalytische Zersetzung des Methanols wird bevorzugt bei einer Temperatur von unter 500°C, besonders bevorzugt von unter 400°C, durchgeführt. Ist für die katalytische Zersetzung von Methanol ein Energieeintrag notwendig, kann dieser durch Nutzung von fossilen Brennstoffen, wie z.B. Erdgas, als Energiequelle, bewirkt werden. Dem erfindungsgemäßen Verfahren wird für die Einstellung der zur katalytischen Spaltung des Methanols notwendigen Temperatur besonders bevorzugt Wärmeenergie zugeführt, die mindestens durch eine Methode bereitgestellt wird, ausgewählt aus (i) der Verbrennung von Brennstoff, der mittels regenerativer Energie erzeugten Wasserstoff (gasförmiges $H_2$) enthält, (ii) der Verbrennung von Brennstoff, der Methan aus biologischer Quelle enthält oder (iii) der Umwandlung von aus regenerativer Energie erzeugter, elektrischer Energie in Wärme. Dabei ist es wiederum besonders bevorzugt, wenn als regenerative Energie wahlweise Windkraft, Sonnenenergie, Wasserkraft oder Mischungen daraus, eingesetzt wird.

Die katalytische Zersetzung des Methanols wird bevorzugt bei einer Temperatur von mindestens 200°C durchgeführt. Dabei ist des wiederum bevorzugt, wenn für die katalytische Zersetzung des Methanols die Temperatur von 200 bis von unter 500°C, besonders bevorzugt von 200 bis unter 400°C eingestellt wird.

Die katalytische Zersetzung des Methanols wird bevorzugt bei einem absoluten Druck von unter 50 bar, besonders bevorzugt von unter 40 bar, durchgeführt.

Der Schritt der katalytischen Zersetzung wird bevorzugt bei einer Temperatur unter 500°C und einem absoluten Druck von unter 50 bar durchgeführt. Besonders bevorzugt wird der Schritt der katalytischen Zersetzung bei einer Temperatur unter 400°C und einem absoluten Druck unter 40 bar durchgeführt. Dabei ist es wiederum ganz besonders bevorzugt, wenn jeweils die Untergrenze der Temperatur von 200°C nicht unterschritten wird.

Das aus der katalytischen Zersetzung erhaltene Produktgas enthält neben Kohlenmonoxid, oftmals zusätzlich Methanol, Kohlendioxid, Wasserstoff und Wasser sowie ggf. Nebenprodukte, welche u.a. aus der Reihe der Ether (Dimethylether), Alkohole (Ethanol), Aldehyde oder Ester stammen können. Vorzugsweise wird das erhaltene Produktgas der katalytischen Zersetzung vor der Umsetzung mit Chlor zu Phosgen in einem bevorzugten, zusätzlichen Prozessschritt einem Reinigungsschritt unterzogen, in dem Methanol, Kohlendioxid, Wasserstoff und Wasser sowie ggf. Nebenprodukte vom Kohlenmonoxid abgetrennt werden.

Das Produktgas der katalytischen Zersetzung wird folglich bevorzugt aufgereinigt und zum Zweck einer Abtrennung von Kohlendioxid einer $CO_2$-Abtrenneinheit zugeführt, bei der das $CO_2$ abgetrennt wird.

Die $CO_2$ Abtrenneinheit und damit die Abtrennung von $CO_2$, kann als "Aminwäsche" ausgeführt werden, wobei das Kohlenmonoxid-haltige Produktgas des Reformerprozesses hier insbesondere die grundsätzlich bekannte Wäsche des Gasgemisches nach dem Prinzip der Chemisorption mit Aminen, wie Monoethanolamin (MEA) Diethanolamin (DEA), Methyldiethanolamin (MDEA) oder Diglycolamin (DGA) durchläuft, welche in einer Absorptionskolonne eine hohe Reinheit des gereinigten Gasgemisches erreicht.

In einer erfindungsgemäß bevorzugten Ausführungsform des Verfahrens erfolgt eine Abtrennung von Wasser aus dem Kohlenmonoxid-haltigen Produktgas.

Im Rahmen einer weiteren bevorzugten Ausführungsform des Verfahrens erfolgt die Abtrennung von Kohlendioxid nachdem zuvor Wasser aus dem Kohlenmonoxid-haltigen Produktgas abgetrennt wurde. Zu diesem Zweck wird das Kohlenmonoxid-haltige Produktgas der katalytischen Zersetzung zunächst einer Wasser-Abtrenneinheit zugeführt, dort das Wasser abgetrennt und das nach der Abtrennung von Wasser erhaltene Kohlenmonoxid-haltige, trockene Produktgas einer $CO_2$-Abtrenneinheit zugeführt, bei der das $CO_2$ abgetrennt wird. In der Wasser-Abtrenneinheit wird zur Abtrennung des Wassers beispielsweise das der katalytischen Zersetzung entnommene Kohlenmonoxid-haltige Produktgas abgekühlt und das Wasser, beispielsweise als Kondensat, abgetrennt.

Als erfindungsgemäß bevorzugte Variante des Verfahrens gilt ein Verfahren, in dem das (bevorzugt vorher von Wasser und von $CO_2$ befreite) Kohlenmonoxid-haltige Produktgas in eine $H_2$-CO Trenneinheit eingebracht wird, in der Wasserstoff abgetrennt wird. Dabei bildet sich zumindest ein Gasstrom, dessen Gas bei 25°C und 1013 mbar mindestens 95 Vol.% Kohlenmonoxid, bevorzugter mindestens 99 Vol.% Kohlenmonoxid, enthält. Das besagte eingebrachte Produktgas wird in der $H_2$-CO Trenneinheit vorzugsweise zunächst in zwei Gasströme aufgetrennt. Es entsteht dabei ein Gas in Form eines Gasstroms, das mindestens 95 Vol.% (bevorzugt mindestens 99 Gew.-%) Kohlenmonoxid enthält, ein weiteres Gas in Form eines Gasstroms, dessen größter Bestandteil Wasserstoff ist und unter anderem Kohlenmonoxid enthält. Das weitere Gas wird auch als Restgas der $H_2$-CO Trennung oder falls keine Restgasbehandlung erfolgt, als Endgas bezeichnet. Eine nach diesem Prinzip der Auftrennung arbeitende $H_2$-CO Trenneinheit ist die sogenannte Coldbox. Das Wasserstoff-haltige Restgas der $H_2$-CO Trennung kann wieder in die Herstellung von Methanol eingebracht werden.

Methanol als nicht reagiertes Edukt sowie Nebenprodukte werden in einer bevorzugten Ausführungsform des Verfahrens aus dem Produktgas der katalytischen Zersetzung entfernt. Bevorzugt werden Edukte und Nebenprodukte in den vorherigen Verfahrensschritt oder weiteren Aufbereitungsschritten zugeführt. Besonders bevorzugt erfolgt die Aufreinigung durch Adsorption oder durch ein thermisches Trennverfahren wie bspw. Destillation bzw. Rektifikation. Besonders bevorzugt werden das Edukt und Nebenprodukte zunächst separiert und die Edukte der Methanolzersetzung zugeführt. Ganz besonders bevorzugt wird der als Produkt anfallende Wasserstoff wie zuvor beschrieben einer Methanolsynthese oder einem Fermentationsprozess zugeführt.

In einer bevorzugten Ausführungsform des Verfahrens wird Wasserstoffgas als Bestandteil des bei der katalytischen Zersetzung erhaltenen Produktgases abgetrennt und für die Bereitstellung von Methanol wiederverwertet. Eine bevorzugte Ausführungsform des Verfahrens kennzeichnet sich daher dadurch, dass

(i) der katalytischen Zersetzung von Methanol solches bereitgestelltes Methanol zugeführt wird, welches Verfahrensprodukt einer Umsetzung von $CO_2$ mit Wasserstoffgas ist, und

(ii) bei der katalytischen Zersetzung des zuvor bereitgestellten Methanols neben Kohlenmonoxid zusätzlich Wasserstoffgas erhalten wird,

mit der Maßgabe, dass bei der katalytischen Zersetzung gebildetes Wasserstoffgas der Umsetzung von $CO_2$ mit Wasserstoffgas zu Methanol zugeführt wird.

Das durch katalytische Zersetzung von Methanol und gegebenenfalls durch Aufreinigung enthaltene Kohlenmonoxid wird in einem nächsten Prozessschritt, bevorzugt über einem Katalysator, zusammen mit Chlor zu Phosgen umgesetzt. Besonders bevorzugt handelt es sich bei dem Katalysator um Aktivkohle.

Das erhaltene Phosgen wird bevorzugt einem zusätzlichen Schritt in Form einer Umsetzung mit mindestens einem organischen Amin oder mit mindestens einer Dihydroxyarylverbindung zugeführt und chemisch umgesetzt. Besonders bevorzugt werden die dabei erhaltenen Produkte einer Reinigung und Aufarbeitung unterzogen.

In einer erfindungsgemäß bevorzugten Umsetzung des Phosgens zur Herstellung eines organischen Isocyanates sind generell solche erfindungsgemäßen Produktionsverfahren bevorzugt, in denen das gewonnene organische Isocyanat mindestens zwei Isocyanatgruppen enthält. Zu diesem Zweck wird bei der Synthese wiederum bevorzugt als Reaktand organisches Amin mit mindestens zwei Aminogruppen eingesetzt.

Ganz besonders bevorzugt eingesetzte organische Amine werden ausgewählt aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4'-diamin oder Mischungen daraus), Hexamethylendiamin, Isophorondiamin, 1,3-Bis(aminomethyl)benzol, Cyclohexyldiamin oder Mischungen daraus, wobei TDA, MDA oder Mischungen daraus ganz besonders bevorzugte organische Amine sind.

Besonders bevorzugt enthält das bei der Umsetzung gewonnene organische Isocyanat mindestens zwei Isocyanatgruppen und weist eine Molmasse von höchstens 1000 g/mol, insbesondere von höchstens 800 g/mol, auf.

Ganz besonders bevorzugt gewonnene organische Isocyanate werden ausgewählt aus Toluylendiisocyanat (TDI), Methylendi(phenylisocyanat) (MDI) (wiederum bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat (HDI), Isophorondiisocyanat (IPDI), 1,3-Bis(isocyanatomethyl)benzol (XDI), Cyclohexyldiisocyanat (CHDI) oder Mischungen daraus, wobei TDI, MDI oder Mischungen daraus ganz besonders bevorzugte organische Isocyanate sind.

Bei der Umsetzung mit organischen Aminen wird besonders bevorzugt ein Gemisch mit dem Hauptbestandteil ausgewählt aus Toluylendiisocyanat, Methylendiphenylisocyanat (bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat, Isophorondiisocyanat, 1,3-Bis(isocyanatomethyl)benzol, oder Cyclohexyldiisocyanat erhalten. Ganz besonders bevorzugt wird im Anschluss an die Umsetzung für eine Reinigung und Aufarbeitung des Hauptbestandteils zumindest der Schritt der Destillation angewendet.

In einer weiteren Ausführungsform des erfindungsgemäßen Verfahrens wird zur Herstellung eines Polycarbonates das Phosgen mit mindestens einer Dihydroxyarylverbindung umgesetzt. Dabei wird die Dihydroxyarylverbindung in einer wässrigen Lösung aus Alkalisalz gelöst. In eine organische Phase wird das Phosgen eingeleitet. Die Umsetzung des Phosgens erfolgt dabei in bekannter Weise mit mindestens einer Dihydroxyarylverbindung, Kohlensäurederivaten, gegebenenfalls Kettenabbrechern und gegebenenfalls Verzweigern, wobei zur Herstellung von Polyestercarbonat ein Teil der Kohlensäurederivate durch aromatische Dicarbonsäuren oder Derivate der Dicarbonsäuren ersetzt wird, und zwar je nach Maßgabe der in den aromatischen Polycarbonaten zu ersetzenden Carbonatstruktureinheiten durch aromatische Dicarbonsäureesterstruktureinheiten.

Sowohl für die Herstellung von Polycarbonat geeignete Dihydroxyarylverbindungen für die Umsetzung mit Phosgen sind solche der Formel (1)

HO-Z-OH (1),

in welcher

Z ein aromatischer Rest mit 6 bis 30 C-Atomen ist, der einen oder mehrere aromatische Kerne enthalten kann, substituiert sein kann und aliphatische oder cycloaliphatische Reste bzw. Alkylaryle oder Heteroatome als Brückenglieder enthalten kann.

Bevorzugt steht Z in Formel (1) für einen Rest der Formel (2)

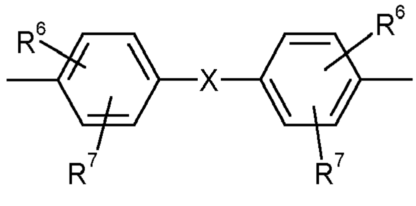

(2),

in der

R6 und R7 unabhängig voneinander für H, C1- bis C18-Alkyl-, C1- bis C18-Alkoxy, Halogen wie Cl oder Br oder für jeweils gegebenenfalls substituiertes Aryl- oder Aralkyl, bevorzugt für H oder C1 bis C12 Alkyl, besonders bevorzugt für H oder C1- bis C8-Alkyl und ganz besonders bevorzugt für H oder Methyl stehen, und

X für eine Einfachbindung, -SO2-, -CO-, -O-, -S-, C1- bis C6-Alkylen, C2- bis C5-Alkyliden oder C5- bis C6 Cycloalkyliden, welches mit C1- bis C6-Alkyl, vorzugsweise Methyl oder Ethyl, substituiert sein kann, ferner für C6- bis C12-Arylen, welches gegebenenfalls mit weiteren Heteroatome enthaltenden aromatischen Ringen kondensiert sein kann, steht.

Bevorzugt steht X für eine Einfachbindung, C1- bis C5-Alkylen, C2- bis C5-Alkyliden, C5- bis C6-Cycloalkyliden, -O-, -SO-, -CO-, -S-, -SO2-
oder für einen Rest der Formel (2a)

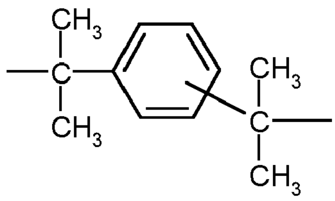

(2a).

Beispiele für Dihydroxyarylverbindungen sind: Dihydroxybenzole, Dihydroxydiphenyle, Bis-(hydroxyphenyl)-alkane, Bis-(hydroxyphenyl)-cycloalkane, Bis-(hydroxyphenyl)-aryle, Bis-(hydroxyphenyl)-ether, Bis-(hydroxyphenyl)-ketone, Bis-(hydroxyphenyl)-sulfide, Bis-(hydroxyphenyl)-sulfone, Bis-(hydroxyphenyl)-sulfoxide, 1,1'-Bis-(hydroxyphenyl)-diisopropylbenzole sowie deren kernalkylierte und kernhalogenierte Verbindungen.

Für die Umsetzung sind geeignete Dihydroxyarylverbindungen beispielsweise Hydrochinon, Resorcin, Dihydroxydiphenyl, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, □,□'-Bis-(hydroxyphenyl)-diisopropylbenzole sowie deren alkylierte, kernalkylierte und kernhalogenierte Verbindungen.

Bevorzugte Dihydroxyarylverbindungen e sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)propan (Bisphenol A (BPA)), 2,4-Bis-(4-hydroxyphenyl)-2-methylbutan, 1,3-Bis-[2-(4-hydroxyphenyl)-2-propyl]-benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan und 1,1-Bis-(4-hydroxyphenyl)-3,3,5-trimethylcyclohexan (Bisphenol TMC (BPTMC)), sowie die Dihydroxyarylverbindungen der Formeln (III) bis (V)

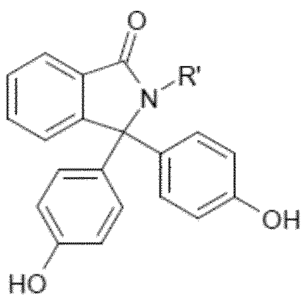

(I)

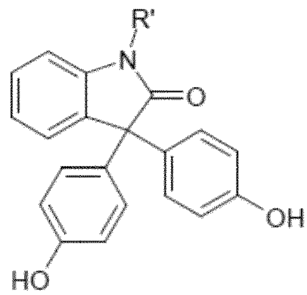

(II)

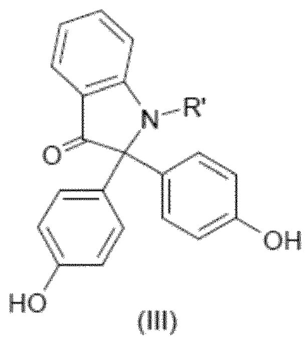

(III)

in denen R' jeweils für C1-C4-Alkyl, Aralkyl oder Aryl, bevorzugt für Methyl oder Phenyl steht.

Besonders bevorzugte Dihydroxyarylverbindungen sind 4,4'-Dihydroxydiphenyl, 1,1-Bis-(4-Hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC (BPTMC)) und die Dihydroxyverbindungen der Formeln (III), (IV) und (V), in denen R' jeweils für C1-C4-Alkyl, Aralkyl oder Aryl, bevorzugt für Methyl oder Phenyl steht.

Diese und weitere geeignete Dihydroxyarylverbindungen sind z.B. in US 2 999 835 A, US 3 148 172 A, US 2 991 273 A, US 3 271 367 A, US 4 982 014 A und US 2 999 846 A, in den deutschen Offenlegungsschriften DE 1 570 703 A1, DE 2 063 050 A1, DE 2 036 052 A1, DE 2 211 956 A1 und DE 3 832 396 A1, der französischen Patentschrift

FR 1 561 518 A1, in der Monographie "H. Schnell, Chemistry and Physics of Polycarbonates, Interscience Publishers, New York 1964, S. 28 ff.; S.102 ff.", und in "D.G. Legrand, J.T. Bendler, Handbook of Polycarbonate Science and Technology, Marcel Dekker New York 2000, S. 72ff." beschrieben.

Im Falle der Umsetzung zu Bereitstellung von Homopolycarbonat wird nur eine Dihydroxyarylverbindung eingesetzt, im Falle von Copolycarbonaten werden zwei oder mehr verschiedene Dihydroxyarylverbindungen eingesetzt. Die verwendete Dihydroxyarylverbindung oder die verwendeten mehreren verschiedenen Dihydroxyarylverbindungen, wie auch alle anderen der Synthese zugesetzten Chemikalien und Hilfsstoffe, können mit den aus ihrer eigenen Synthese, Handhabung und Lagerung stammenden Verunreinigungen kontaminiert sein. Es ist jedoch wünschenswert, mit möglichst reinen Rohstoffen zu arbeiten.

In der gleichen Weise werden eventuell zu verwendende Verzweiger oder Verzweigermischungen der Synthese zugesetzt. Üblicherweise werden Trisphenole, Quarterphenole oder Säurechloride von Tri- oder Tetracarbonsäuren verwendet oder auch Gemische der Polyphenole oder der Säurechloride.

Das eingesetzte Alkalisalz zur Bildung des Alkalisalzes der Dihydroxyarylverbindungen kann eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.%-ige Lösung eingesetzt.

Die Reaktion zwischen Phosgen und dem Alkalisalz der Dihydroxyarylverbindung kann durch Katalysatoren, wie tertiäre Amine, N-Alkylpiperidine oder Oni-umsalze beschleunigt werden. Bevorzugt werden Tributylamin, Triethylamin und N-Ethylpiperidin verwendet.

Der eingesetzte Amin-Katalysator kann offenkettig oder zyklisch sein, besonders bevorzugt ist Triethylamin und Ethylpiperidin. Der Katalysator wird im neuen Verfahren vorzugsweise als 1 bis 55 Gew.%-ige Lösung eingesetzt.

Unter Oniumsalzen werden hier Verbindungen wie NR4X verstanden, wobei R ein Alkyl und/oder Arylrest und/oder ein H sein kann und X ein Anion ist.

Die Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, isoOctylphenol, Cumylphenol, deren Chlorkohlensäureester oder Säurechloride von Monocar-bonsäuren bzw. Gemischen aus diesen Kettenabbrechern. Bevorzugte Kettenabbrecher sind Phenol, Cumyl-phenol, Isooctylphenol, para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Bei-spiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenyl-methan, 2,2-Bis-[4,4-bis-(4-hydroxyphenyl)-cyclohexyl]-propan, 2,4-Bis-(4-hydroxyphenyl-iso¬propyl)-phenol, Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydro-indol und 1,1,1-Tri-(4-hydroxyphenyl)-ethan.

Es kann für die Bereitstellung von Polycarbonat ebenso zunächst aus dem erfindungsgemäß erhaltenen Phosgen Diphenylcarbonat hergestellt und anschließend mindestens eine Dihydroxyarylverbindung mit Diphenylcarbonat umgesetzt werden.

Ein weiterer Gegenstand der Erfindung ist die Einbindung des o.g. Verfahrens in eine Prozesskette zur Produktion von Kohlenmonoxid. Bevorzugt wird das Verfahren in Kombination mit einem Verfahren ausgewählt aus der Reihe Dampfreformierung, RWGS oder $CO_2$ Elektrolyse betrieben.

Ein weiterer Gegenstand der Erfindung ist die diskontinuierliche Durchführung des o.g. Verfahrens, dadurch gekennzeichnet, dass die katalytische Spaltung von Methanol flexibel betrieben wird. Dabei wird Methanol in einem Zwischenspeicher (bspw. ein Tank) bevorratet und bei Bedarf in den o.g. Prozess eingespeist. Besonders bevorzugt wird diese Fahrweise in Kombination mit einem weiteren Verfahren, ausgewählt aus der Reihe Dampfreformierung, RWGS oder $CO_2$ Elektrolyse, betrieben. Die Kombination wird dabei so gestaltet, dass alle Verfahren flexibel gefahren werden können.

Ein weiterer Gegenstand der Erfindung ist die Verwendung von als Verfahrensprodukt einer katalytischen Zersetzung von Methanol bereitgestelltem Kohlenmonoxid zur Herstellung von Phosgen.

Für diesen Gegenstand gelten die vorgenannten bevorzugten Ausführungsformen der für das Verfahren zur Herstellung des bereitgestellten Kohlenmonoxides definierten Merkmale mutatis mutandis, insbesondere die für die Herstellung des Methanols und der katalytischen Zersetzung des Methanols genannten Merkmale.

Ein weiterer Gegenstand der Erfindung ist ein Mehrkomponenten-System zur Herstellung von Phosgen aus Chlor und Kohlenmonoxid, zumindest enthaltend

i) als eine erste Komponente Kohlenmonoxid als Verfahrensprodukt aus einer katalytischen Zersetzung von Methanol und

ii) als eine zweite Komponente eine Vorrichtung, mindestens umfassend

- mindestens eine Zuleitung, welche die erste Komponente enthält, und
- mindestens eine Vorrichtung zur Herstellung von Phosgen, die mindestens einen in Fluidverbindung mit der besagten Zuleitung stehenden Einlass für Kohlenmonoxid aufweist, sowie mindestens einen Einlass für Chlor und mindestens einen Auslass für Phosgen enthält.

Unter einer Fluidverbindung wird eine Vorrichtung verstanden, die Vorrichtungsteile miteinander verbindet und durch die sich ein Stoff, der in jedem Aggregatzustand vorliegen kann, als Stoffstrom von einem Vorrichtungsteil zum nächsten transportieren lässt, beispielsweise eine Zuleitung in Form eines Rohres. Der Begriff "in Fluidverbindung stehen" bedeutet, dass benannte Vorrichtungsteile über eine Fluidverbindung miteinander verbunden sind.

Ein "Reaktionsraum" ist ein Volumen einer Vorrichtung, in dem die an einer chemischen Reaktion beteiligten Reaktionspartner zusammen vorliegen und in dem die chemische Reaktion stattfindet. Dies kann für eine chemische Reaktion beispielsweise das Volumen eines Gefäßes oder Behälters sein, in dem sich die Reaktionspartner gemeinsam befinden und zur Reaktion gebracht werden sollen.

Bevorzugte Ausführungsformen der Bereitstellung der ersten Komponente werden bereits bei der Darstellung des erfindungsgemäßen Verfahrens genannt und gelten für das Mehrkomponenten-System mutatis mutandis.

Im Rahmen einer bevorzugten Ausführungsform des Mehrkomponenten-Systems steht die die erste Komponente enthaltende mindestens eine Zuleitung zusätzlich in Fluidverbindung mit einer Vorrichtung zur Herstellung der ersten Komponente, wobei die Vorrichtung zur Herstellung der ersten Komponente mindestens einen Reaktionsraum mit mindestens einem Einlass für Methanol, mindestens einem Katalysator und mindestens einem Auslass für ein die erste Komponente enthaltendes Produktgas enthält, wobei der Auslass für das besagte Produktgas, gegebenenfalls über eine Vorrichtung zur Reinigung, mit der die erste Komponente enthaltenden mindestens einen Zuleitung der zweiten Komponente in Fluidverbindung steht. Bevorzugt enthält die Vorrichtung zur Herstellung der ersten Komponente zusätzlich mindestens Heizelement als eine Vorrichtung zur Zuführung von Wärmeenergie zum Reaktionsraum und/oder zur Verdampfung des Methanols. Dabei ist es wiederum besonders bevorzugt, wenn das Heizelement in Fluidverbindung mit mindestens einer Quelle, ausgewählt aus einer Quelle für aus regenerativer Energie bereitgestelltem Wasserstoff als Brennstoff oder einer Quelle für aus biologischer Quelle bereitgestelltem Methan als Brennstoff, steht oder das Heizelement an eine Quelle zur Bereitstellung von elektrischer Energie aus regenerativer Energie für die Umwandlung in Wärmeenergie angeschlossen ist. Dabei ist es wiederum besonders bevorzugt, wenn als regenerative Energie wahlweise Windkraft, Sonnenenergie, Wasserkraft, Bioenergie oder Mischungen daraus, eingesetzt wird.

Der in der Vorrichtung zur Herstellung der ersten Komponente bevorzugt enthaltene Katalysator wird wie im zuvor beschriebenen Verfahren ausgewählt.

Es hat sich als vorteilhaft herausgestellt, wenn mindestens ein Einlass für Methanol der Vorrichtung zur Herstellung der ersten Komponente in Fluidverbindung mit einer Methanol-Quelle steht, in der Methanol enthalten ist, welches aus der Umsetzung der Komponenten eines Gasstroms erhalten wurde, der mindestens $CO_x$ mit x = 1 oder 2 und bevorzugt zusätzlich Wasserstoffgas enthält.

Dafür steht der Einlass für Methanol in Fluidverbindung mit einem Lagerbehälter, in dem das entsprechende Methanol gelagert wird oder der Einlass für Methanol steht in Fluidverbindung mit einer Vorrichtung zur Methanolherstellung. Entsprechende Vorrichtungen zur Methanolherstellung sind dem Fachmann aus dem Stand der Technik bekannt und lassen sich am Markt kaufen. Dies sind bevorzugt Vorrichtungen zur Dampfreformierung von Kohlenwasserstoffen (insbesondere von Methan), Trockenreformierung von Methan, zu partiellen Oxidation von Kohlenwasserstoffen, zur Vergasung von Biomasse, zur Elektrolyse, zur Durchführung des Reverse Water Gas Shifts.

Das Methanol der Methanol-Quelle wird in einer besonders bevorzugten Ausführungsform durch (i) eine Vorrichtung zur katalytischen Umsetzung eines Synthesegases, enthaltend mehr als 50 Vol.% einer Mischung aus Kohlendioxid und Wasserstoff, (ii) durch eine Fermentationsvorrichtung oder (iii) durch eine Vorrichtung zur elektrochemischen Umwandlung von Kohlendioxid, hergestellt. Dabei wird jeder Vorrichtung (i) bis (iii) ein $CO_2$ haltiger Stoffstrom als Feed über einen Einlass zugeführt. Im Falle der Fermentationsvorrichtung (ii) und insb. der Vorrichtung zur katalytischen Umsetzung (i) enthält der Feed zudem Wasserstoff.

Besonders bevorzugt wird der für die Schritte (i) oder (ii) benötigte Wasserstoff durch mindestens ein Verfahren, ausgewählt aus der Elektrolyse von Wasser, der partiellen Oxidation oder der Biomassevergasung, oder Mischungen davon hergestellt. In diesem Zusammenhang ist es besonders bevorzugt, wenn die Vorrichtung zur Methanolherstellung mindestens einen Einlass aufweist, der in Fluidverbindung dem Auslass von Wasserstoffgas einer Elektrolysevorrichtung steht. Dabei ist es besonders bevorzugt, wenn die Stromzufuhr der Elektrolysevorrichtung mit einer Quelle von aus regenerativer Energie hergestelltem elektrischen Strom in Kontakt steht. Dabei ist es wiederum besonders bevorzugt,

wenn als regenerative Energie wahlweise Windkraft, Sonnenenergie, Wasserkraft, Bioenergie oder Mischungen daraus, eingesetzt wird.

Im Rahmen einer besonders bevorzugten Ausführungsform steht die die erste Komponente enthaltende mindestens eine Zuleitung des Mehrkomponenten-Systems zusätzlich in Fluidverbindung mit einer Vorrichtung zur Herstellung der ersten Komponente, wobei die Vorrichtung zur Herstellung der ersten Komponente mindestens einen Einlass für Methanol, mindestens einen Katalysator und mindestens einen Auslass für ein die erste Komponente enthaltendes Produktgas enthält, wobei der Auslass für das besagte Produktgas der Vorrichtung zur Herstellung der ersten Komponente über eine Vorrichtung zur Reinigung mit der mindestens einen, die erste Komponente enthaltenden Zuleitung der zweiten Komponente in Fluidverbindung steht und die Vorrichtung zur Reinigung mindestens einen Einlass für besagtes Produktgas, mindestens einen Auslass für abgetrenntes Methanol, mindestens einen Auslass für abgetrenntes Wasserstoffgas und mindestens einen Auslass für die erste Komponente enthält und der Auslass für die erste Komponente mit der mindestens einen, die erste Komponente enthaltenden Zuleitung der zweiten Komponente in Fluidverbindung steht.

Im Rahmen dieser besonders bevorzugten Ausführungsform des Mehrkomponenten-Systems ist eine Ausführungsform erfindungsgemäß ganz besonders bevorzugt, worin der Auslass für Wasserstoffgas der Vorrichtung zur Reinigung mit einer Vorrichtung zur Hydrierung, bevorzugt mit einer Vorrichtung zur Hydrierung von Kohlendioxid zu Methanol als eine Vorrichtung zur Methanolherstellung, in Fluidverbindung steht.

Im Rahmen der besonders bevorzugten Ausführungsform des Mehrkomponenten-Systems umfassend eine Fluidverbindung über eine Vorrichtung zur Reinigung ist eine Ausführungsform erfindungsgemäß ganz besonders bevorzugt, worin der Auslass für Methanol der Vorrichtung zur Reinigung mit der Vorrichtung zur katalytischen Zersetzung von Methanol in Fluidverbindung steht.

Eine weitere bevorzugte Ausführungsform des Mehrkomponenten-Systems ist dadurch gekennzeichnet, dass die Vorrichtung zur Herstellung der ersten Komponente mindestens eine Vorrichtung zur Verdampfung des Methanols enthält.

Eine weitere bevorzugte Ausführungsform des Mehrkomponenten-Systems ist dadurch gekennzeichnet, dass die Vorrichtung zur Herstellung der ersten Komponente mindestens ein Heizelement als Vorrichtung zur Einbringung von Wärmeenergie in den Reaktionsraum und/oder in die Vorrichtung zur Verdampfung des Methanols enthält.

Die Nutzung des durch das Mehrkomponenten-System produzierte Phosgens erfolgt bevorzugt wie zuvor beschrieben durch einen weiteren Schritt, in dem das in der Vorrichtung zur Herstellung von Phosgen gebildete Phosgen einer Umsetzung mit mindestens einem organischen Amin oder mit mindestens einer Dihydroxyarylverbindung zugeführt und chemisch umgesetzt wird. Aus diesem Grund ist es bevorzugt, wenn mindestens ein Auslass für Phosgen der Vorrichtung zur Herstellung von Phosgen in Fluidverbindung mit mindestens einer Vorrichtung zur Phosgenierung von organischem Amin oder mit mindestens einer Vorrichtung zur Herstellung von Polycarbonat steht. Entsprechende Vorrichtungen zur Phosgenierung aromatischer Amine oder zur Herstellung von Polycarbonat sind dem Fachmann aus dem Stand der Technik bekannt.

Das Verfahren und bevorzugte Ausführungsformen davon werden beispielhaft in den Figuren Fig. 1 und Fig.2 illustriert, ohne den Erfindungsgegenstand darauf zu beschränken. Pfeile in den Figuren symbolisieren den Fluss von Stoffen, Energie oder Wärme zwischen Verfahrensschritten bzw. Vorrichtungsteilen, in denen die entsprechenden Verfahrensschritte ablaufen. Gestrichelte Linien in den Figuren bedeuten Teile von bevorzugten Ausführungsformen einzelner zuvor beschriebener Merkmale des Verfahrens. Strich-Punkt Rahmen veranschaulichen den Bereich der Herkunft der für das erfindungsgemäße Verfahren bereitgestellten Rohstoffe, hier Methanol und Kohlenmonoxid. In den Figuren werden folgende Bezugszeichen verwendet:

1 Herstellung von Phosgen aus Chlor und Kohlenmonoxid

2 Bereitgestelltes Kohlenmonoxid, welches ein Verfahrensprodukt einer katalytischen Zersetzung von Methanol (4) ist

3 Phosgen

4 katalytische Zersetzung von Methanol

5 Bereitgestelltes Methanol, welches ein Verfahrensprodukt einer Umsetzung 6, ausgewählt aus einer elektrochemischen Reaktion, einer homogen katalysierten Reaktion oder einer heterogen katalysierten Reaktion ist

6 Herstellung von Methanol durch Umsetzung eines Gasstroms mit $CO_x$ worin x = 1 oder 2 ist und mit gegebenenfalls Wasserstoff (bevorzugt von mindestens $CO_2$ und Wasserstoff), wobei das hergestellte Methanol ein Verfahrensprodukt einer Umsetzung, ausgewählt aus einer elektrochemischen Reaktion, einer homogen katalysierten Reaktion oder einer heterogen katalysierten Reaktion ist

7 elektrischer Strom aus regenerativer Energie

8 Elektrolyse von Wasser

9 Isocyanat Herstellung und/oder Polycarbonat Herstellung

10 Bereich der Herkunft des bereitgestellten Kohlenmonoxids 2

11 Bereich der Herkunft des bereitgestellten Methanols 5

12 Lagerung von Methanol, welches ein Verfahrensprodukt der Herstellung 6 ist

Fig.1 zeigt eine Ausführungsform eines erfindungsgemäßen Verfahrens, in der Kohlenmonoxid **2**, welches ein Verfahrensprodukt einer katalytischen Zersetzung von Methanol **4** ist, bereitgestellt wird, z.B. durch Entnahme von entsprechendem Methanol aus einem Lagertank oder aus einer Leitung, die in Fluidverbindung mit der katalytischen Zersetzung von Methanol **4** steht. Dieses bereitgestellte Kohlenmonoxid **2** wird in eine Herstellung von Phosgen **1** geleitet, wo es mit Chlor ($Cl_2$) zu Phosgen **3** umgesetzt und aus der Herstellung von Phosgen **1** herausgeführt wird, bevorzugt gefolgt von einer Zuführung des Phosgens **3** in eine Isocyanat Herstellung **9**. Das in Fig.1 bereitgestellte Methanol **5** ist bevorzugt ein Verfahrensprodukt einer Herstellung von Methanol **6** durch Umsetzung von Wasserstoff mit mindestens $CO_2$, wobei das hergestellte Methanol ein Verfahrensprodukt einer Umsetzung, ausgewählt aus einer elektrochemischen Reaktion, einer homogen katalysierten Reaktion oder einer heterogen katalysierten Reaktion ist. Dabei ist es wiederum besonders bevorzugt, wenn Wasserstoff genutzt wird, der mittels Elektrolyse **8** von Wasser unter Einsatz von elektrischem Strom aus regenerativer Energie **7** wurde. Der Bereich **10** der Herstellung des bereitgestellten Kohlenmonoxids **2** markiert entsprechende Verfahrensschritte bzw. Anlagenteile, die für die Bereitstellung vom Lieferanten oder dem Phosgenproduzenten selbst durchgeführt werden. Der Bereich **11** der Herstellung des bereitgestellten Methanols **5** markiert entsprechende Verfahrensschritte bzw. Anlagenteile, die für die Bereitstellung von einem Lieferanten des bereitgestellten Kohlenmonoxids **2**, von einem weiteren Lieferanten von bereitgestelltem Methanol **5** oder dem Phosgenproduzenten selbst durchgeführt werden können.

Fig.2 zeigt eine Ausführungsform eines erfindungsgemäßen Verfahrens, in der Kohlenmonoxid **2**, welches ein Verfahrensprodukt einer katalytischen Zersetzung von Methanol **4** ist, durch Entnahme von entsprechendem Methanol aus einem Lagertank **12**, der in Fluidverbindung mit der katalytischen Zersetzung von Methanol **4** steht, in eine Herstellung von Phosgen **1** geleitet wird, wo es mit Chlor ($Cl_2$) zu Phosgen **3** umgesetzt und aus der Herstellung von Phosgen **1** herausgeführt wird, bevorzugt gefolgt von einer Zuführung des Phosgens **3** in eine Isocyanat Herstellung **9**. Bei der Zersetzung von Methanol **4** wird Wasserstoffgas freigesetzt, welches der Herstellung von Methanol **6** zugeführt wird. Die Herstellung von Methanol **6** findet durch Umsetzung von Wasserstoff mit mindestens $CO_2$ statt, wobei das hergestellte Methanol ein Verfahrensprodukt einer Umsetzung, ausgewählt aus einer elektrochemischen Reaktion, einer homogen katalysierten Reaktion oder einer heterogen katalysierten Reaktion ist. Falls die Menge an aus der Zersetzung von Methanol **4** anfallendem Wasserstoffgas nicht für die Herstellung von Methanol **6** ausreicht, ist es besonders bevorzugt, wenn zusätzlicher Wasserstoff genutzt wird, der mittels Elektrolyse **8** von Wasser unter Einsatz von elektrischem Strom aus regenerativer Energie **7** erzeugt wird.

Im Rahmen von Ausführungsformen der Erfindung sind beispielsweise die nachfolgenden Aspekte 1 bis 22 zu nennen:

1. Verfahren zur Herstellung von Phosgen aus Chlor und Kohlenmonoxid (1), dadurch gekennzeichnet, dass durch katalytische Zersetzung von Methanol (4) erhaltenes Kohlenmonoxid bereitgestellt und dieses bereitgestellte Kohlenmonoxid (2) mit Chlor zu Phosgen (3) umgesetzt wird.

2. Verfahren nach Aspekt 1, dadurch gekennzeichnet, dass vor der katalytischen Zersetzung von Methanol (4) solches Methanol (5) bereitgestellt wird, welches Verfahrensprodukt einer Umsetzung (6), ausgewählt aus einer elektrochemischen Reaktion, einer homogen katalysierten Reaktion oder einer heterogen katalysierten Reaktion ist.

3. Verfahren nach Aspekt 1 oder 2, dadurch gekennzeichnet, dass vor der katalytischen Zersetzung von Methanol (4) solches Methanol (5) bereitgestellt wird, welches Verfahrensprodukt einer Umsetzung (6) eines Gasstroms ist, der mindestens $CO_x$ mit $x = 1$ oder 2 und gegebenenfalls Wasserstoff, bevorzugt $CO_2$ und Wasserstoff, enthält..

4. Verfahren nach Aspekt 1 oder 2, dadurch gekennzeichnet, dass vor der katalytischen Zersetzung von Methanol (4) Methanol bereitgestellt wird, welches Verfahrensprodukt einer Umsetzung eines Kohlenmonoxid und Wasserstoff haltigem Gasstroms ist, der durch eine Methode oder einer Kombination von Methoden aus der Reihe Dampfreformierung von Methan, partielle Oxidation von Kohlenwasserstoffen, Vergasung von Biomasse, Elektrolyse, Reverse Water Gas Shift erhalten wird.

5. Verfahren nach einem der Aspekte 3 oder 4, dadurch gekennzeichnet, dass der Wasserstoff des Wasserstoff haltigen Gasstroms durch Elektrolyse von Wasser (8) unter Verwendung von elektrischem Strom aus regenerativer Energie (7) erhalten wird.

6. Verfahren nach einem der vorgehenden Aspekte, dadurch gekennzeichnet, dass zur Bereitstellung des Methanols (5) dieses aus einem Lagerbehälter (12) entnommen und/oder im Rahmen eines kontinuierlichen Verfahrens vorab

durch mindestens einen Prozess der Aspekte 2 bis 4 hergestellt und jeweils der katalytischen Zersetzung (4) zugeführt wird.

7. Verfahren nach einem der Aspekte 1 bis 6, dadurch gekennzeichnet, dass in einem weiteren Schritt das gebildete Phosgen (3) einer Umsetzung (9) mit mindestens einem organischen Amin zugeführt wird, wobei ein Produktgemisch mit einem Hauptbestandteil ausgewählt aus Toluylendiisocyanat, Methylendiphenylisocyanat (bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat, Isophorondiisocyanat, 1,3-Bis(isocyanatomethyl)benzol, oder Cyclohexyldiisocyanat erhalten wird.

8. Verfahren nach einem der Aspekte 1 bis 6, dadurch gekennzeichnet, dass in einem weiteren Schritt das gebildete Phosgen (3) einer Umsetzung (9) mit mindestens einer Dihydroxyarylverbindung zugeführt wird, wobei ein Polycarbonat erhalten wird.

9. Verfahren nach einem der vorangehenden Aspekte, dadurch gekennzeichnet, dass zur katalytischen Zersetzung von Methanol (4) das Methanol (5) bei einer Temperatur von unter 500°C, insbesondere von unter 400°C, katalytisch zersetzt wird.

10. Verfahren nach einem der vorangehenden Aspekte, dadurch gekennzeichnet, dass zur katalytischen Zersetzung von Methanol (4) das Methanol (5) bei einem absoluten Druck von unter 50 bar, insbesondere unter 40 bar, katalytisch zersetzt wird.

11. Verfahren nach einem der vorangehenden Aspekte, dadurch gekennzeichnet, dass zur katalytischen Zersetzung (4) bereitgestelltes Methanol (5) als gasförmiges Methanol mit einem Katalysator in Kontakt gebracht und katalytisch unter Bildung von Kohlenmonoxid (2) und gegebenenfalls zusätzlich von Wasserstoffgas zersetzt wird.

12. Verfahren nach einem der vorangehenden Aspekte, dadurch gekennzeichnet, dass zur katalytischen Zersetzung von Methanol (4) gasförmiges Methanol über einen Katalysator, dessen Aktivkomponente mindestens eine Übergangsmetall-Spezies enthält, geleitet wird.

13. Verfahren nach einem der vorangehenden Aspekte, dadurch gekennzeichnet, dass zur katalytischen Zersetzung des Methanols (5) ein Katalysator eingesetzt wird, der mindestens eine auf einen Träger aufgebrachte Übergangsmetall-Spezies, insbesondere mindestens eine auf einen Träger aufgebrachte Übergangsmetall-Spezies ausgewählt aus der Gruppe VII, VIII oder XI des Periodensystems der Elemente, enthält.

14. Verfahren nach einem der vorangehenden Aspekte, dadurch gekennzeichnet, dass

(i) der katalytischen Zersetzung von Methanol (4) bereitgestelltes Methanol (5) zugeführt wird, welches Verfahrensprodukt einer Umsetzung von $CO_2$ mit Wasserstoffgas ist, und

(ii) bei der katalytischen Zersetzung (4) des zuvor bereitgestellten Methanols (5) neben Kohlenmonoxid (2) zusätzlich Wasserstoffgas erhalten wird,
mit der Maßgabe, dass bei der katalytischen Zersetzung (4) gebildetes Wasserstoffgas der Umsetzung von $CO_2$ mit Wasserstoffgas zu Methanol (5) zugeführt wird.

15. Mehrkomponenten-System zur Herstellung von Phosgen aus Chlor und Kohlenmonoxid, zumindest enthaltend

i) als eine erste Komponente Kohlenmonoxid, erhalten als Verfahrensprodukt aus einer katalytischen Zersetzung von Methanol und

ii) als eine zweite Komponente eine Vorrichtung, mindestens umfassend

- mindestens eine Zuleitung, welche die erste Komponente enthält, und
- mindestens eine Vorrichtung zur Herstellung von Phosgen, die mindestens einen in Fluidverbindung mit der besagten Zuleitung stehenden Einlass für Kohlenmonoxid aufweist, sowie mindestens einen Einlass für Chlor und mindestens einen Auslass für Phosgen enthält.

16. Mehrkomponenten-System nach Aspekt 15, dadurch gekennzeichnet, dass die die erste Komponente enthaltende mindestens eine Zuleitung zusätzlich in Fluidverbindung mit einer Vorrichtung zur Herstellung der ersten Komponente steht, wobei die Vorrichtung zur Herstellung der ersten Komponente mindestens einen Einlass für Methanol, mindestens einen Katalysator und mindestens einen Auslass für ein die erste Komponente enthaltendes Produktgas enthält, wobei der Auslass für das besagte Produktgas, gegebenenfalls über eine Vorrichtung zur Reinigung, mit der die erste Komponente enthaltenden mindestens einen Zuleitung der zweiten Komponente in Fluidverbindung steht.

17. Mehrkomponenten-System nach Aspekt 16, dadurch gekennzeichnet, dass der Auslass für das Produktgas der Vorrichtung zur Herstellung der ersten Komponente über eine Vorrichtung zur Reinigung mit der mindestens einen, die erste Komponente enthaltenden Zuleitung der zweiten Komponente in Fluidverbindung steht, und die Vorrichtung zur Reinigung mindestens einen Einlass für besagtes Produktgas, mindestens einen Auslass für abgetrenntes Methanol, mindestens einen Auslass für abgetrenntes Wasserstoffgas und mindestens einen Auslass für die erste Komponente enthält und der Auslass für die erste Komponente mit der mindestens einen, die erste Komponente enthaltenden Zuleitung der zweiten Komponente in Fluidverbindung steht.

18. Mehrkomponenten-System nach Aspekt 17, dadurch gekennzeichnet, dass der Auslass für Wasserstoffgas der Vorrichtung zur Reinigung mit einer Vorrichtung zur Hydrierung, bevorzugt mit einer Vorrichtung zur Hydrierung von Kohlendioxid zu Methanol als eine Vorrichtung zur Methanolherstellung, in Fluidverbindung steht.

19. Mehrkomponenten-System nach einem der Aspekte 16 bis 18, dadurch gekennzeichnet, dass der mindestens ein Einlass für Methanol der Vorrichtung zur Herstellung der ersten Komponente in Fluidverbindung mit einer Methanol-Quelle steht, in der Methanol enthalten ist, welches aus der Umsetzung der Komponenten eines Gasstroms erhalten wurde, der mindestens $CO_x$ mit x = 1 oder 2 und bevorzugt zusätzlich Wasserstoffgas enthält.

20. Mehrkomponenten-System nach einem der Aspekte 15 bis 19, dadurch gekennzeichnet, dass der Katalysator der Vorrichtung zur Herstellung der ersten Komponente ausgewählt wird, aus mindestens einem Übergangsmetall-Spezies ausgewählt aus der Gruppe VII, VIII oder XI des Periodensystems der Elemente, welches bevorzugt auf einen Träger aufgebracht ist.

21. Mehrkomponenten-System nach einem der Aspekte 15 bis 20, dadurch gekennzeichnet, dass die Vorrichtung zur Herstellung der ersten Komponente mindestens ein Heizelement als Vorrichtung zur Einbringung von Wärmeenergie in den Reaktionsraum und/oder in eine Vorrichtung zur Verdampfung von Methanol enthält.

22. Mehrkomponenten-System nach einem der Aspekte 15 bis 21, dadurch gekennzeichnet, dass mindestens ein Auslass für Phosgen der Vorrichtung zur Herstellung von Phosgen in Fluidverbindung mit mindestens einer Vorrichtung zur Phosgenierung von organischem Amin oder mit mindestens einer Vorrichtung zur Herstellung von Polycarbonat steht.

23. Verwendung von als Verfahrensprodukt einer katalytischen Zersetzung von Methanol bereitgestelltem Kohlenmonoxid zur Herstellung von Phosgen.

## Patentansprüche

1. Verfahren zur Herstellung von Phosgen aus Chlor und Kohlenmonoxid (1), **dadurch gekennzeichnet, dass** durch katalytische Zersetzung von Methanol (4) erhaltenes Kohlenmonoxid bereitgestellt und dieses bereitgestellte Kohlenmonoxid (2) mit Chlor zu Phosgen (3) umgesetzt wird.

2. Verfahren nach Anspruch 1, **dadurch gekennzeichnet, dass** vor der katalytischen Zersetzung von Methanol (4) solches Methanol (5) bereitgestellt wird, welches Verfahrensprodukt einer Umsetzung (6), ausgewählt aus einer elektrochemischen Reaktion, einer homogen katalysierten Reaktion oder einer heterogen katalysierten Reaktion ist.

3. Verfahren nach Anspruch 1 oder 2, **dadurch gekennzeichnet, dass** vor der katalytischen Zersetzung von Methanol (4) solches Methanol (5) bereitgestellt wird, welches Verfahrensprodukt einer Umsetzung (6) eines Gasstroms ist, der mindestens $CO_x$ mit x = 1 oder 2 und gegebenenfalls Wasserstoff, bevorzugt $CO_2$ und Wasserstoff, enthält..

4. Verfahren nach Anspruch 1 oder 2, **dadurch gekennzeichnet, dass** vor der katalytischen Zersetzung von Methanol (4) Methanol (5) bereitgestellt wird, welches Verfahrensprodukt einer Umsetzung eines Kohlenmonoxid und Wasserstoff haltigem Gasstroms ist, der durch eine Methode oder einer Kombination von Methoden aus der Reihe Dampfreformierung von Methan, partielle Oxidation von Kohlenwasserstoffen, Vergasung von Biomasse, Elektrolyse, Reverse Water Gas Shift erhalten wird.

**5.** Verfahren nach einem der Ansprüche 3 oder 4, **dadurch gekennzeichnet, dass** der Wasserstoff des Wasserstoff haltigen Gasstroms durch Elektrolyse von Wasser (8) unter Verwendung von elektrischem Strom aus regenerativer Energie (7) erhalten wird.

**6.** Verfahren nach einem der vorgehenden Ansprüche, **dadurch gekennzeichnet, dass** zur Bereitstellung des Methanols (5) dieses aus einem Lagerbehälter entnommen und/oder im Rahmen eines kontinuierlichen Verfahrens vorab durch mindestens einen Prozess der Ansprüche 2 bis 4 hergestellt und jeweils der katalytischen Zersetzung (4) zugeführt wird.

**7.** Verfahren nach einem der Ansprüche 1 bis 5, **dadurch gekennzeichnet, dass** in einem weiteren Schritt das gebildete Phosgen (3) einer Umsetzung (9) mit mindestens einem organischen Amin zugeführt wird, wobei ein Produktgemisch mit einem Hauptbestandteil ausgewählt aus Toluylendiisocyanat, Methylendiphenylisocyanat (bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat, Isophorondiisocyanat, 1,3-Bis(isocyanatomethyl)benzol, oder Cyclohexyldiisocyanat erhalten wird.

**8.** Verfahren nach einem der Ansprüche 1 bis 4, **dadurch gekennzeichnet, dass** in einem weiteren Schritt das gebildete Phosgen (3) einer Umsetzung (9) mit mindestens einer Dihydroxyarylverbindung zugeführt wird, wobei ein Polycarbonat erhalten wird.

**9.** Verfahren nach einem der vorangehenden Ansprüche, **dadurch gekennzeichnet, dass** zur katalytischen Zersetzung von Methanol (4) das Methanol (5) bei einer Temperatur unter 500°C, insbesondere unter 400°C, katalytisch zersetzt wird.

**10.** Verfahren nach einem der vorangehenden Ansprüche, **dadurch gekennzeichnet, dass** zur katalytischen Zersetzung von Methanol (4) das Methanol (5) bei einem absoluten Druck von unter 50 bar, insbesondere unter 40 bar, katalytisch zersetzt wird.

**11.** Verfahren nach einem der vorangehenden Ansprüche, **dadurch gekennzeichnet, dass** zur katalytischen Zersetzung (4) bereitgestelltes Methanol (5)als gasförmiges Methanol mit einem Katalysator in Kontakt gebracht und katalytisch unter Bildung von Kohlenmonoxid (2) und gegebenenfalls zusätzlich von Wasserstoffgas zersetzt wird.

**12.** Verfahren nach einem der vorangehenden Ansprüche, **dadurch gekennzeichnet, dass** zur katalytischen Zersetzung von Methanol (4) gasförmiges Methanol über einen Katalysator, dessen Aktivkomponente mindestens eine Übergangsmetall-Spezies enthält, geleitet wird.

**13.** Verfahren nach einem der vorangehenden Ansprüche, **dadurch gekennzeichnet, dass** zur katalytischen Zersetzung von Methanol (4) ein Katalysator eingesetzt wird, der mindestens eine auf einen Träger aufgebrachte Übergangsmetall-Spezies, insbesondere mindestens eine auf einen Träger aufgebrachte Übergangsmetall-Spezies ausgewählt aus der Gruppe VII, VIII oder XI des Periodensystems der Elemente, enthält.

**14.** Verfahren nach einem der vorangehenden Ansprüche, **dadurch gekennzeichnet, dass**

(i) der katalytischen Zersetzung von Methanol (4) bereitgestelltes Methanol (5) zugeführt wird, welches Verfahrensprodukt einer Umsetzung (6) von $CO_2$ mit Wasserstoffgas ist, und
(ii) bei der katalytischen Zersetzung (4) des zuvor bereitgestellten Methanols (5) neben Kohlenmonoxid (2) zusätzlich Wasserstoffgas erhalten wird,
mit der Maßgabe, dass bei der katalytischen Zersetzung (4) gebildetes Wasserstoffgas der Umsetzung von $CO_2$ mit Wasserstoffgas zu Methanol (5) zugeführt wird.

**15.** Mehrkomponenten-System zur Herstellung von Phosgen aus Chlor und Kohlenmonoxid, zumindest enthaltend

i) als eine erste Komponente Kohlenmonoxid, erhalten als Verfahrensprodukt aus einer katalytischen Zersetzung von Methanol und

ii) als eine zweite Komponente eine Vorrichtung, mindestens umfassend

- mindestens eine Zuleitung, welche die erste Komponente enthält, und
- mindestens eine Vorrichtung zur Herstellung von Phosgen, die mindestens einen in Fluidverbindung mit der besagten Zuleitung stehenden Einlass für Kohlenmonoxid aufweist, sowie mindestens einen Einlass für Chlor und mindestens einen Auslass für Phosgen enthält.

**16.** Mehrkomponenten-System nach Anspruch 15, **dadurch gekennzeichnet, dass** die die erste Komponente enthaltende mindestens eine Zuleitung zusätzlich in Fluidverbindung mit einer Vorrichtung zur Herstellung der ersten Komponente steht, wobei die Vorrichtung zur Herstellung der ersten Komponente mindestens einen Einlass für Methanol, mindestens einen Katalysator und mindestens einen Auslass für ein die erste Komponente enthaltendes Produktgas enthält, wobei der Auslass für das besagte Produktgas, gegebenenfalls über eine Vorrichtung zur Reinigung, mit der die erste Komponente enthaltenden mindestens einen Zuleitung der zweiten Komponente in Fluidverbindung steht.

**17.** Mehrkomponenten-System nach Anspruch 15 oder 16, **dadurch gekennzeichnet, dass** der Katalysator der Vorrichtung zur Herstellung der ersten Komponente ausgewählt wird, aus mindestens einem Übergangsmetall-Spezies ausgewählt aus der Gruppe VII, VIII oder XI des Periodensystems der Elemente, welches bevorzugt auf einen Träger aufgebracht ist.

**18.** Mehrkomponenten-System nach einem der Ansprüche 15 bis 17, **dadurch gekennzeichnet, dass** mindestens ein Auslass für Phosgen der Vorrichtung zur Herstellung von Phosgen in Fluidverbindung mit mindestens einer Vorrichtung zur Phosgenierung von organischem Amin oder mit mindestens einer Vorrichtung zur Herstellung von Polycarbonat steht.

**19.** Verwendung von als Verfahrensprodukt einer katalytischen Zersetzung von Methanol bereitgestelltem Kohlenmonoxid zur Herstellung von Phosgen.

Fig.1

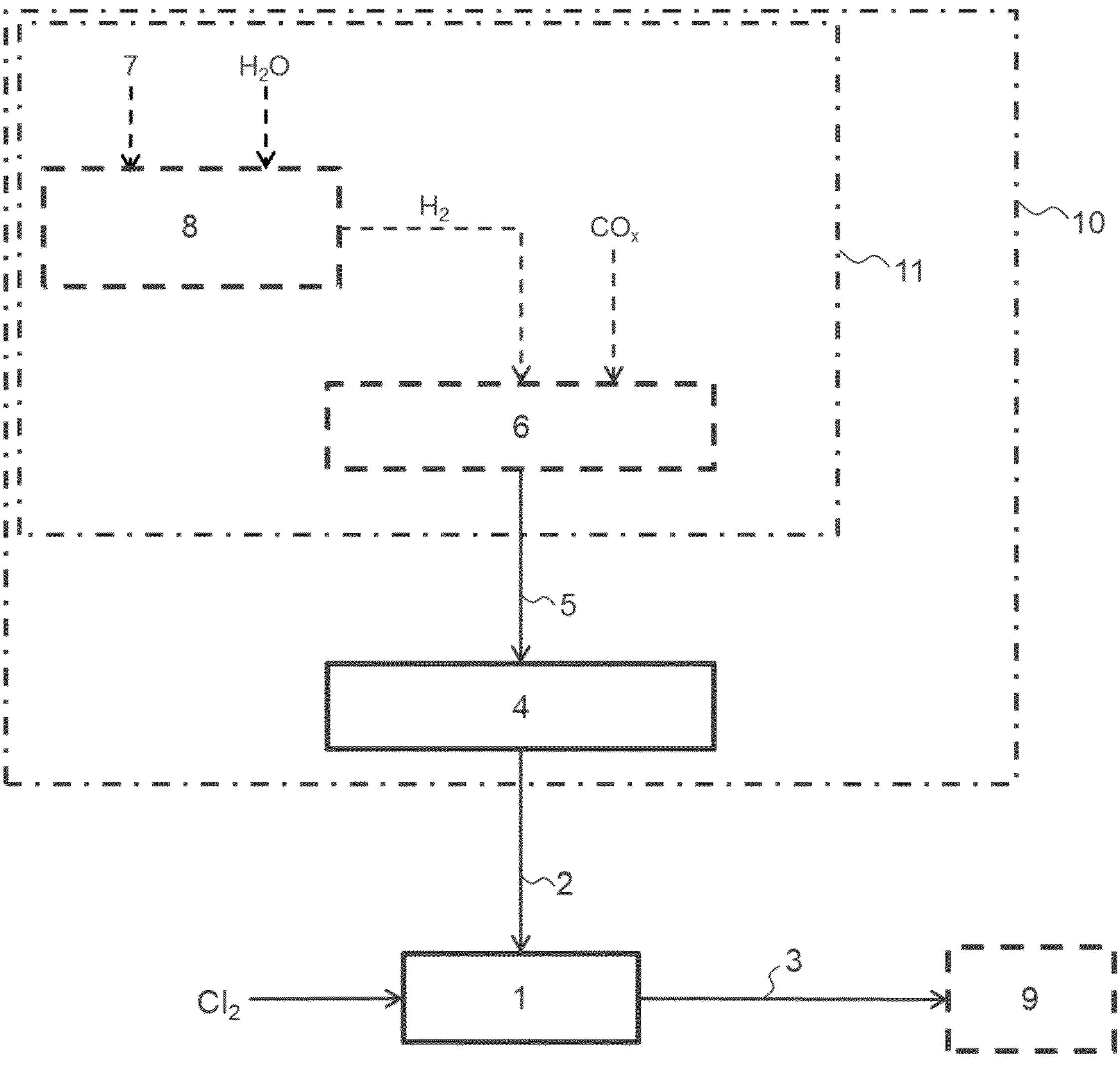

**Fig.2**

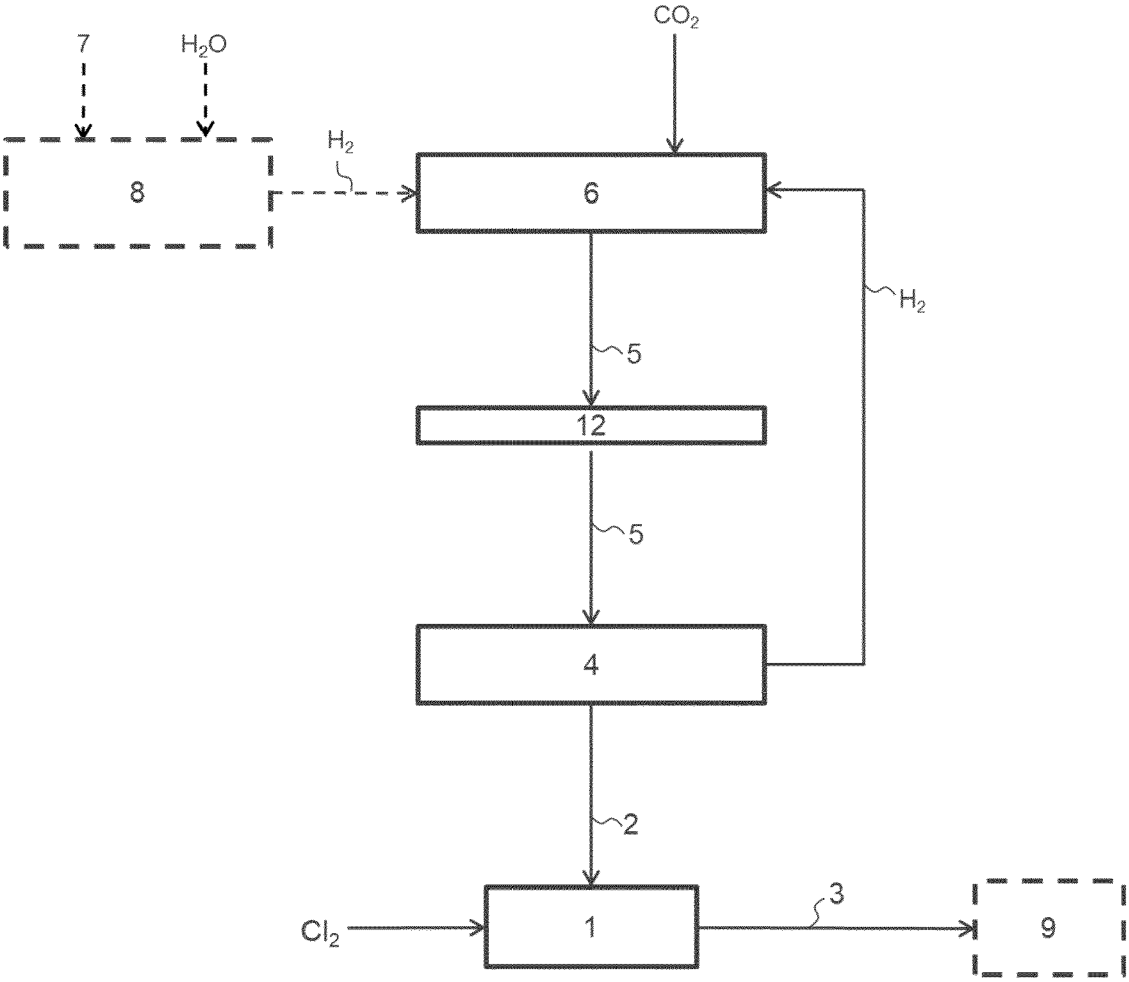

Europäisches Patentamt
European Patent Office
Office européen des brevets

# EUROPÄISCHER RECHERCHENBERICHT

Nummer der Anmeldung
EP 21 20 1336

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2006/183938 A1 (GRABOWSKI STEFAN [DE] ET AL) 17. August 2006 (2006-08-17)<br>* Absätze [0013] - [0016]; Ansprüche 1,9 *<br>* das ganze Dokument *<br>----- | 1-19 | INV.<br>C01B32/80<br>C01B3/22<br>C01B3/26<br>C07C1/04<br>C10K3/02<br>C07C263/10 |
| X | DE 103 11 865 A1 (BASF AG [DE]) 30. September 2004 (2004-09-30)<br>* Anspruch 1; Abbildung 1 *<br>* das ganze Dokument *<br>----- | 1-19 | |
| X | EP 0 846 713 A2 (MITSUBISHI CHEM CORP [JP]) 10. Juni 1998 (1998-06-10)<br>* (a) Phosgene; (b) Diphenol;<br>Seite 2, Zeilen 1-56; Ansprüche 5, 8, 9 *<br>* das ganze Dokument *<br>----- | 1-19 | |
| X | DATABASE WPI<br>Week 199132<br>Thomson Scientific, London, GB;<br>AN 1991-233811<br>XP002805951,<br>-& JP H03 151337 A (MITSUI TOATSU CHEM INC) 27. Juni 1991 (1991-06-27)<br>* Zusammenfassung *<br>* citations refer to the machine translation into English;<br>Absatz [0001] *<br>* das ganze Dokument *<br>----- | 1-14,19 | RECHERCHIERTE SACHGEBIETE (IPC)<br>C01B<br>C01C<br>C07C<br>C10K |

Der vorliegende Recherchenbericht wurde für alle Patentansprüche erstellt

| Recherchenort | Abschlußdatum der Recherche | Prüfer |
|---|---|---|
| Den Haag | 17. März 2022 | Straub, Thomas |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 21 20 1336

In diesem Anhang sind die Mitglieder der Patentfamilien der im obengenannten europäischen Recherchenbericht angeführten Patentdokumente angegeben. Die Angaben über die Familienmitglieder entsprechen dem Stand der Datei des Europäischen Patentamts am Diese Angaben dienen nur zur Unterrichtung und erfolgen ohne Gewähr.

17-03-2022

| Im Recherchenbericht angeführtes Patentdokument | Datum der Veröffentlichung | Mitglied(er) der Patentfamilie | Datum der Veröffentlichung |
|---|---|---|---|
| US 2006183938 A1 | 17-08-2006 | AT 465987 T | 15-05-2010 |
| | | BR PI0600382 A | 03-10-2006 |
| | | CN 1821213 A | 23-08-2006 |
| | | DE 102005006692 A1 | 24-08-2006 |
| | | EP 1690851 A1 | 16-08-2006 |
| | | ES 2343197 T3 | 26-07-2010 |
| | | JP 4868498 B2 | 01-02-2012 |
| | | JP 2006225385 A | 31-08-2006 |
| | | KR 20060092085 A | 22-08-2006 |
| | | PT 1690851 E | 25-06-2010 |
| | | SG 125202 A1 | 29-09-2006 |
| | | TW 200640838 A | 01-12-2006 |
| | | US 2006183938 A1 | 17-08-2006 |
| DE 10311865 A1 | 30-09-2004 | KEINE | |
| EP 0846713 A2 | 10-06-1998 | DE 69728842 T2 | 21-04-2005 |
| | | EP 0846713 A2 | 10-06-1998 |
| | | KR 19980063929 A | 07-10-1998 |
| | | US 5986037 A | 16-11-1999 |
| JP H03151337 A | 27-06-1991 | JP 2764080 B2 | 11-06-1998 |
| | | JP H03151337 A | 27-06-1991 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0517044 A2
- EP 520272 A2
- EP 1882681 A
- US 6730285 B2
- US 6340437 B1
- US 6583084 B
- EP 0409517 A
- US 20050108941 A
- US 6699457 B
- EP 3489394 A
- US 2999835 A
- US 3148172 A
- US 2991273 A
- US 3271367 A
- US 4982014 A
- US 2999846 A
- DE 1570703 A1
- DE 2063050 A1
- DE 2036052 A1
- DE 2211956 A1
- DE 3832396 A1
- FR 1561518 A1
- DE 3833953 A


### In der Beschreibung aufgeführte Nicht-Patentliteratur


- **W. SIEFKEN.** *Liebigs Ann.,* 1949, vol. 562, 75-106
- *Ullmanns Encyklopädie der technischen Chemie,* 1977, vol. 13, 351-353
- **G. WEGENER.** Applied Catalysis A: General. Elsevier Science, 2001, vol. 221, 303-335
- Chemistry and Physics of Polycarbonates. **VON SCHNELL.** Polymer Reviews. Interscience Publishers, 1964, vol. 9, 33-70
- Polycarbonates. **D. FREITAG ; U. GRIGO ; P.R. MÜLLER ; N. NOUVERTNE ; BAYER AG.** Encyclopedia of Polymer Science and Engineering. 1988, vol. 1 1, 651-692
- Ullmann's Encyclopedia of Industrial Chemistry. VCH Verlagsgesellschaft mbH, 1991, vol. A, 19
- **MITCHELL et al.** Selection of carbon catalysts for the industrial manufacture of phosgene. *Catal. Sci. Technol.,* 2012, vol. 2, 2109-2115
- Ullmann Enzyklopädie der technischen Chemie. 1975, vol. 9, 357
- Winnacker-Küchler: Chemische Technik, Prozesse und Produkte. Wiley-VCH Verlag, 2005, vol. 3, 512
- *Ullmann Encyclopedia of industrial chemistry,* 2006
- Gas production. *ULLMANN'S Encyclopedia of Industrial Chemistry,* 2016
- **T. HAAS.** *Nature Catalysis,* 2018, vol. 1, 32-39
- **DITTMEYER et al.** Chemische Technik. vol. 4
- *ChemCatChem,* 2019, vol. 11, 4238-4246
- *Topics in Catalysis,* April 2003, vol. 22 (3—4
- **VOLKMAR M. SCHMIDT.** Elektrochemische Verfahrenstechnik. Wiley-VCH-Verlag, 2003
- **H. SCHNELL.** Chemistry and Physics of Polycarbonates. Interscience Publishers, 1964, 28, , 102
- **D.G. LEGRAND ; J.T. BENDLER.** Handbook of Polycarbonate Science and Technology. Marcel Dekker, 2000, 72